# EUROPEAN PATENT APPLICATION

(11) **EP 4 851 173 A1**
(43) Date of publication of application: **09.12.2026**
(21) Application number: 25748176.2
(22) Date of filing: 28.01.2025
(51) Int. Cl.: C07F 19/00, C07C 53/08, C07C 53/124, C07C 61/135, C07F 17/02, B01J 31/24

(54) **RUTHENIUM-DIPHOSPHINE-CARBOXYLATE COMPLEX AND METHOD FOR PRODUCING SAME**

(30) Priority: 29.01.2024 JP 2024011084
(71) Applicant: Takasago International Corporation, Tokyo 144-8721 (JP)
(72) Inventor: NAKAYAMA, Yuji, Hiratsuka-shi, Kanagawa 254-0073 (JP); SEKI, Mizuki, Hiratsuka-shi, Kanagawa 254-0073 (JP); HARABE, Takumi, Hiratsuka-shi, Kanagawa 254-0073 (JP); SAKAGUCHI, Kazuhiko, Hiratsuka-shi, Kanagawa 254-0073 (JP); TAMAKI, Nao, Hiratsuka-shi, Kanagawa 254-0073 (JP)
(74) Representative: Hoffmann Eitle
(86) International application number: PCT/JP2025/002530
(87) International publication number: WO 2025/164594

(57) **Abstract**

The present invention relates to a novel ruthenium complex useful as a catalyst in various organic synthesis reactions, including aromatic asymmetric hydrogenation, and an efficient method for producing the same. Namely, the present invention provides a ruthenium-diphosphine-carboxylate complex represented by general formula (1). The present invention further provides a method for producing a ruthenium-diphosphine-carboxylate complex represented by general formula (1), the method comprising reacting a diphosphine compound represented by general formula (2) with a ruthenium-alkylbenzene-carboxylate complex represented by general formula (3).

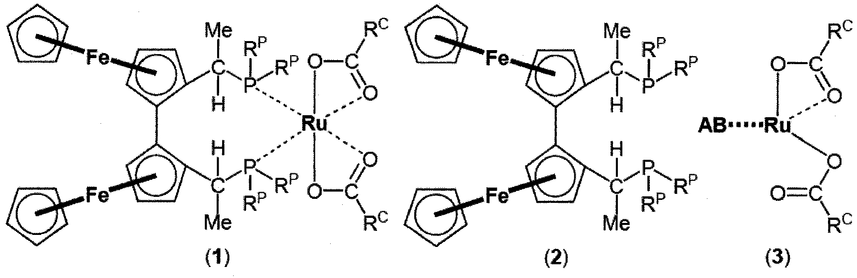

## Description

### Technical Field

The present invention relates to a novel ruthenium-diphosphine-carboxylate complex useful as a catalyst for various organic synthesis reactions, including the asymmetric hydrogenation of aromatics, and an efficient method for producing the same.

### Background Art

Optically active cyclic compounds are of great importance as pharmaceuticals, agrochemicals, functional materials, fragrances, and synthetic intermediates. Accordingly, intensive research and development efforts continue to be directed toward their production methods. Among such methods, the catalytic asymmetric hydrogenation of aromatic compounds or heteroaromatic compounds, referred to as the asymmetric hydrogenation of aromatics, offers several advantages, including the ability to simultaneously introduce multiple chiral carbon centers into a target molecule, as well as excellent atom economy and the potential for significant reduction of waste. Therefore, the asymmetric hydrogenation of aromatics is not only useful as the method for producing optically active cyclic compounds, but has also become one of the particularly important catalytic reactions from the perspectives of the Sustainable Development Goals (SDGs), which have been attracting attention recently, and Green Chemistry, which contributes to reducing environmental impact.

For the practical application of the asymmetric hydrogenation of aromatics, it is essential to employ chiral catalysts that are not only easy to produce but also exhibit excellent catalytic activity, asymmetric induction ability, substrate generality, reaction reproducibility, and air stability. Accordingly, intensive efforts have been made over many years to develop such catalysts. Among them, the catalyst systems prepared by combining 2,2"-bis[1-(diphenylphosphino)ethyl]-1,1"-biferrocene (commonly known as Ph-TRAP, Patent Literature 1 and Non-Patent Literature 1), a rare trans-chelating diphosphine compound, with various transition metal sources are known to exhibit excellent asymmetric induction ability and substrate generality in the asymmetric hydrogenation of aromatics (Non-Patent Literatures 2 to 4). Therefore, extensive research has also been conducted on the synthesis methods of Ph-TRAP, which is used to prepare such catalyst systems, and various methods have been reported to date (Non-Patent Literatures 5 to 7).

On the other hand, the desired reactions are not always be reproducible in the aforementioned catalyst systems. These problems arise because the complex formation between Ph-TRAP and the transition metal sources does not always proceed smoothly in the reaction systems (Non-Patent Literature 2). In order to solve these problems, Ryoichi Kuwano and his colleagues at Kyushu University developed a ruthenium complex of Ph-TRAP, namely [RuCl(p-cymene)(Ph-trap)]Cl. They demonstrated that the reaction reproducibility was significantly improved by preparing and isolating this complex outside the reaction system before using it as a catalyst (Non-Patent Literatures 2 and 8). Furthermore, the asymmetric hydrogenation of indoles, naphthalenes, and quinolines by using this ruthenium complex as a catalyst has enabled the efficient synthesis of optically active indolines, which are important as pharmaceutical and agrochemical intermediates, as well as optically active tetrahydronaphthalenes and tetrahydroquinolines, which are difficult to obtain by conventional methods (Non-Patent Literatures 8 to 10). For reference, Formula 1 below shows the stereochemical structures of both enantiomers of [RuCl(p-cymene)(Ph-trap)]Cl; Formula 2 below shows a schematic diagram of the asymmetric hydrogenation of aromatics catalyzed by this complex.

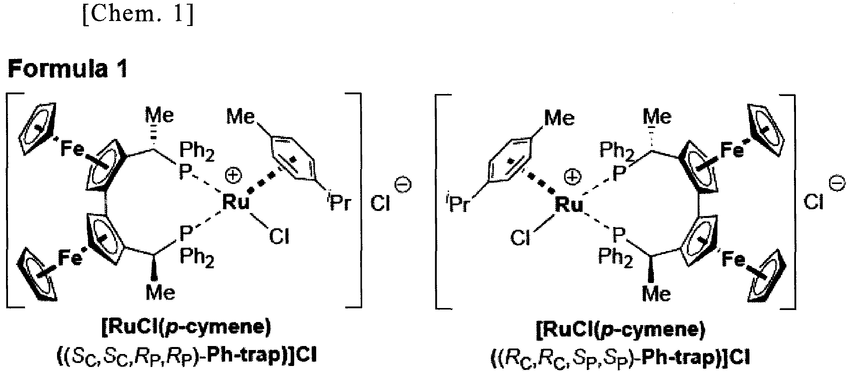

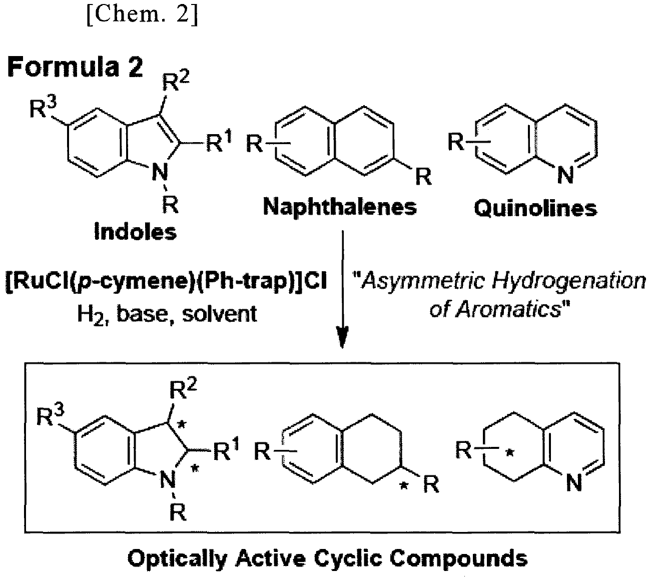

### Citation List

### Patent Literature

Patent Literature 1: JP-A-H04-283596

### Non-Patent Literature

Non-Patent Literature 1: Masaya Sawamura, Hitoshi Hamashima, and Yoshihiko Ito, Tetrahedron Asymmetry, 1991, 7(2), 593-596.
Non Patent Literature 2: Ryoichi Kuwano, J. Synth. Org. Chem., Jpn., 2007, 65(2), 109-118.
Non-Patent Literature 3: Ryoichi Kuwano, Nao Kameyama, and Ryuhei Ikeda, J. Am. Chem. Soc., 2011, 133(19), 7312-7315.
Non-Patent Literature 4: Ryoichi Kuwano, J. Synth. Org. Chem., Jpn., 2021, 79(12), 1125-1135.
Non-Patent Literature 5: Masaya Sawamura, Hitoshi Hamashima, Masanobu Sugawara, Ryoichi Kuwano, and Yoshihiko Ito, Organometallics, 1995, 14(10), 4549-4558.
Non-Patent Literature 6: Ryoichi Kuwano and Masaya Sawamura, Catalysts for Fine Chemical Synthesis: Vol. 5, Regio- and Stereo- Controlled Oxidations and Reductions, Wiley, Chichester, 2007, 73-86.
Non-Patent Literature 7: Michael A. Schmidt, Eric M. Simmons, Carolyn S. Wei, Hyunsoo Park, and Martin D. Eastgate, J. Org. Chem., 2018, 83(7), 3928-3940.
Non-Patent Literature 8: Ryoichi Kuwano and Manabu Kashiwabara, Org. Lett., 2006, 8(12), 2653-2655.
Non-Patent Literature 9: Ryoichi Kuwano, Ryuichi Morioka, Manabu Kashiwabara, and Nao Kameyama, Angew. Chem. Int. Ed., 2012, 51(17), 4136-4139.
Non-Patent Literature 10: Ryoichi Kuwano, Ryuhei Ikeda, and Kazuki Hirasada, Chem. Commun., 2015, 51, 7558-7561.

### Summary of Invention

### Technical Problem

To date, various catalysts have been vigorously developed to improve the efficiency of the asymmetric hydrogenation of aromatics, which is useful for producing optically active cyclic compounds, and various organic synthesis reactions. On the other hand, the aforementioned SDGs and Green Chemistry cannot necessarily be achieved through the application of conventional catalysts, and therefore, new catalyst systems are always in high demand. The objective of the present invention is to provide a new catalyst that can be suitably used in various organic synthesis reactions, and an efficient method for producing the same.

### Solution to Problem

From the perspective of developing such new catalysts, the ruthenium complex of Ph-TRAP mentioned above, namely [RuCl(p-cymene)(Ph-trap)]Cl, should be re-examined. While this complex exhibits excellent performance as a catalyst for the asymmetric hydrogenation of aromatics, various problems remain from the standpoint of industrial applications, as described below. Specifically, the synthesis of Ph-TRAP, which is used to produce this complex, requires various reagents and solvents that are difficult to use industrially. Furthermore, the product forms of Ph-TRAP either become an amorphous substance that is unstable in air, or a highly toxic benzene solvate. Therefore, the industrial production of Ph-TRAP by the conventional methods is extremely difficult (Formula 3; Non-Patent Literature 5 to 7).

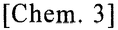

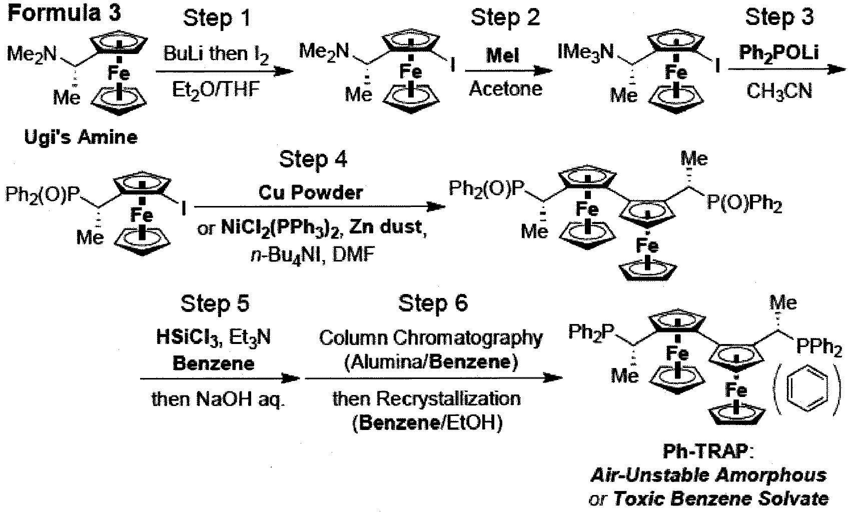

The desired [RuCl(p-cymene)(Ph-trap)]Cl can be synthesized by the reaction of the dichloro(p-cymene)ruthenium(II) dimer ([RuCl₂(p-cymene)]₂) with Ph-TRAP obtained by the method described above. However, this reaction requires an excess amount of dichloromethane, a halogenated solvent subject to strict legal regulations. In addition, the poor crystallinity and amorphous nature of this complex make its industrial production difficult (Formula 4; Non-Patent Literature 8).

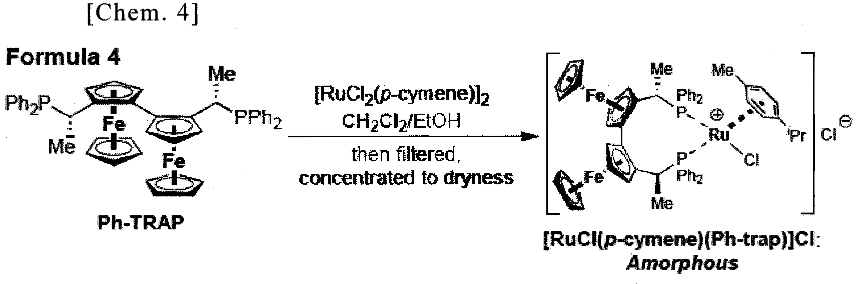

Furthermore, while the asymmetric hydrogenation of aromatics catalyzed by this complex has the advantage of not requiring highly toxic or corrosive fluorine-containing solvents commonly used in similar reactions, it instead requires the coexistence of various strong bases, such as potassium carbonate, cesium carbonate, or 1,1,3,3-tetramethylguanidine (TMG), making it difficult to use compounds that are prone to decompose under basic conditions as substrates. In addition, since this reaction also requires more than 1 mol% of the catalyst, there is room for further improvement of this complex (Formula 5; Non-Patent Literatures 8 to 10).

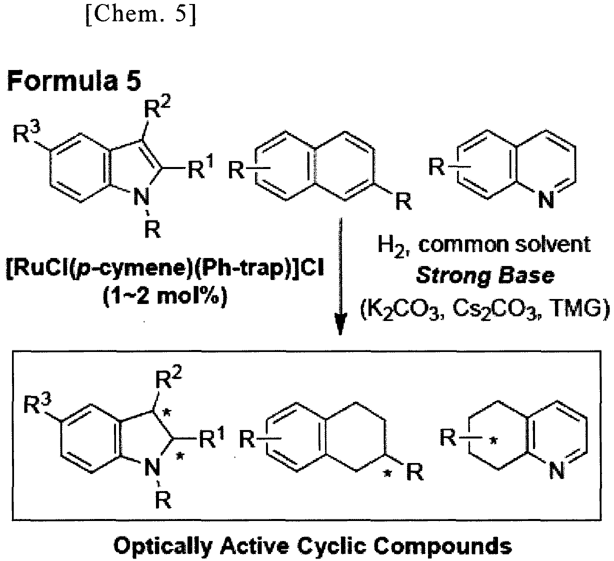

In light of the foregoing background, the inventors initiated research aimed at the industrialization of synthetically challenging Ph-TRAP. As a result, a method was developed to efficiently produce a stable equivalent of Ph-TRAP (Ph-TRAP·(BH₃)₂) while avoiding various problems in the conventional methods (International Publication No. WO2024/203802). Furthermore, the inventors also discovered that this stable equivalent can be readily converted to Ph-TRAP·n-BuOH, a crystalline solvate that is stable even in air, simply by heating and cooling it in n-butyl alcohol (n-BuOH) (International Publication No. WO2024/203803). For reference, Formula 6 below shows the outline of these methods for producing Ph-TRAP·(BH₃)₂ and Ph-TRAP·n-BuOH described above (the present invention is not limited in any way by this outline).

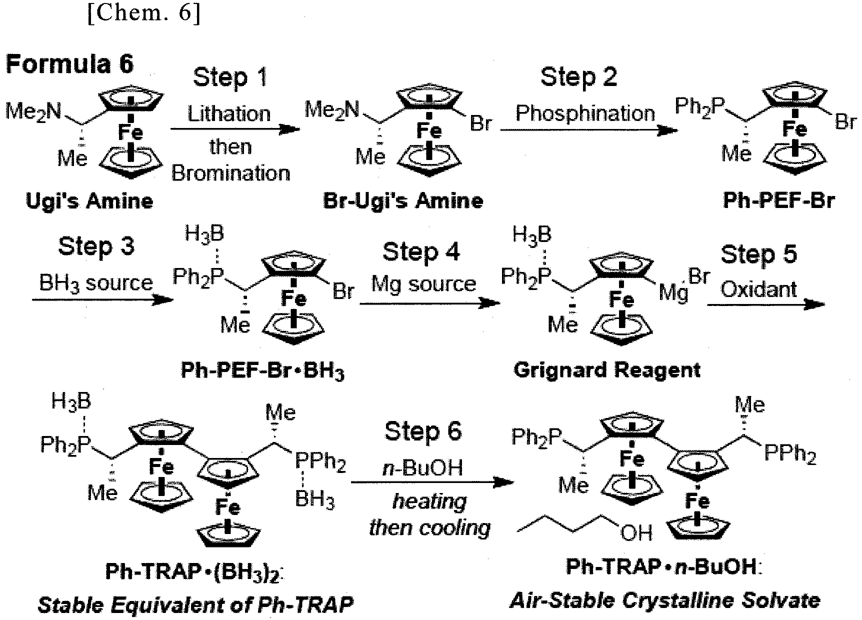

These production methods made it possible to obtain Ph-TRAP, which was previously difficult to synthesize, in abundant quantities as a stable crystalline solvate. Therefore, the inventors focused on the molecular design of a new catalyst in order to solve the problems of the conventional catalyst, namely [RuCl(p-cymene)(Ph-trap)]Cl. As a result, the inventors established a molecular design that is expected to exhibit not only improved crystallinity due to enhanced molecular symmetry, but also excellent catalytic activity without the addition of strong bases, by substantially removing the p-cymene and chloride ligands from the conventional catalyst and instead introducing acetate ligands that function as a weak base (Formula 7).

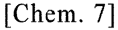

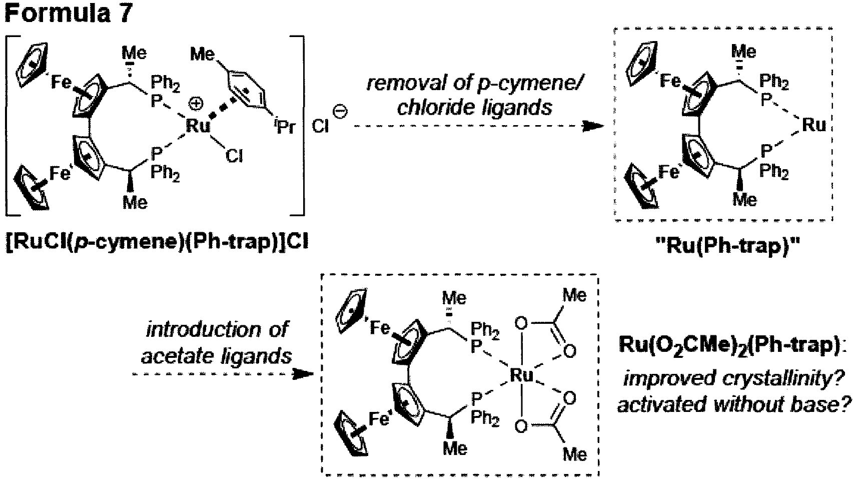

Based on this strategic molecular design, the inventors diligently pursued research aimed at developing a new catalyst. As a result, a method was developed to produce a novel ruthenium-diphosphine-acetate complex, namely Ru(O₂CMe)₂(Ph-trap), as a crystalline compound in high yield by reacting Ph-TRAP·n-BuOH with the known ruthenium-alkylbenzene-acetate complex, namely diacetate(p-cymene)ruthenium(II) (Ru(O₂CMe)₂(p-cymene)), using toluene as a non-halogenated solvent. Furthermore, by utilizing its excellent crystallinity, the absolute configuration of this complex was successfully determined by single-crystal X-ray structural analysis. Surprisingly, the analysis revealed that Ph-TRAP in this complex functions as a cis-chelating chiral diphosphine ligand, rather than the conventional trans-chelating ligand. For reference, Formula 8 below shows the outline of the method for producing Ru(O₂CMe)₂(Ph-trap) (the present invention is not limited in any way by this outline).

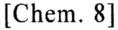

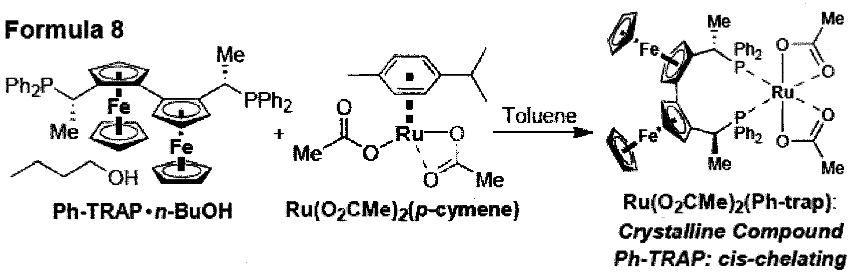

Next, the inventors carried out the asymmetric hydrogenation of N-Boc-2-phenyl-1H-indole, a type of bulky, unreactive heteroaromatic compound, as a model reaction to evaluate the performance of various catalysts. As a result, when using the conventional [RuCl(p-cymene)(Ph-trap)]Cl or common Ru(O₂CMe)₂(binap) as a catalyst, the model reaction hardly proceeded without the addition of a base. On the other hand, when using the newly developed Ru(O₂CMe)₂(Ph-trap) as a catalyst, the desired reaction was completed rapidly without the addition of a base, and the amount of the catalyst required could be reduced to as little as 0.05 mol%. Furthermore, the desired N-Boc-2-phenylindoline was quantitatively yielded with extremely high optical purity in this hydrogenation. For reference, Formula 9 below shows the outline of the asymmetric hydrogenation of N-Boc-2-phenyl-1H-indole by using various catalysts (the present invention is not limited in any way by this outline).

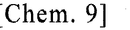

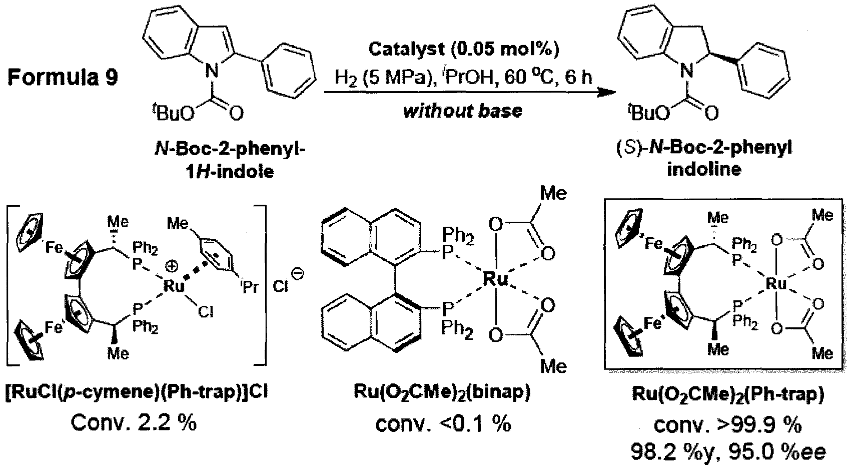

Furthermore, the catalytic activity of Ru(O₂CMe)₂(Ph-trap) was significantly improved along with a change in molecular symmetry by replacing the acetate ligand (O₂CMe) on the complex with bulkier carboxylate ligands such as a pivalate ligand (O₂C^{t}Bu) or a 1-adamantanecarboxylate ligand (O₂CAd), enabling the asymmetric hydrogenation to be completed even using more practical low-pressure hydrogen gas.

Building upon these findings, the inventors conducted further studies and completed the present invention. Specifically, the present invention includes the following [1] to [11].
[1] A ruthenium-diphosphine-carboxylate complex represented by the following general formula (1). 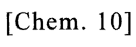 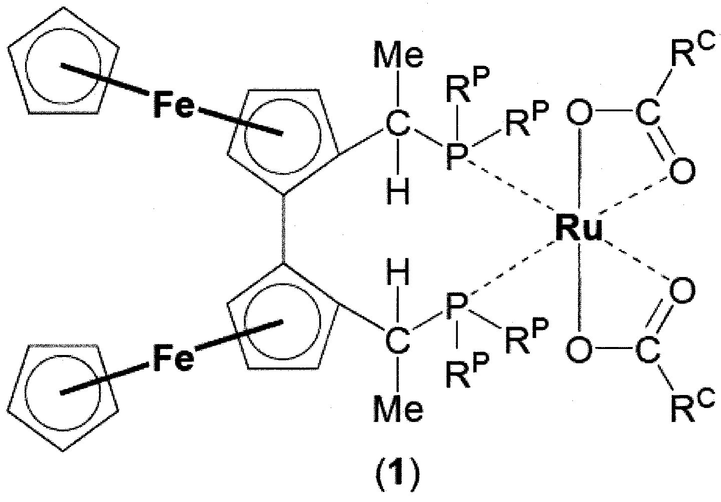 [Wherein,
   a solid line represents a single bond, a double line represents a double bond, and a dashed line represents a coordinate bond;
   H represents a hydrogen atom, C represents a carbon atom, O represents an oxygen atom, and P represents a phosphorus atom;
   Me represents a methyl group;
   Fe represents a divalent iron ion, a pentagon containing a circle represents a cyclopentadienyl anion, and a bold line represents six-electron donation of the cyclopentadienyl anion to Fe;
   R^{P} represents a group selected from the group consisting of an alkyl group, a cycloalkyl group, a heteroaryl group, and an aryl group which may have a substituent;
   Ru represents a divalent ruthenium ion;
   R^{C} represents a group selected from the group consisting of an alkyl group, a halogenoalkyl group, a cycloalkyl group, and an aryl group.]
[2] The ruthenium-diphosphine-carboxylate complex according to the above [1], which is an optically active form.
[3] The ruthenium-diphosphine-carboxylate complex according to the above [1] or [2], wherein R^{P} is an aryl group which may have a substituent.
[4] The ruthenium-diphosphine-carboxylate complex according to any one of the above [1] to [3], wherein R^{C} is selected from the group consisting of an alkyl group and a cycloalkyl group.
[5] A method for producing the ruthenium-diphosphine-carboxylate complex according to any one of the above [1] to [4], comprising reacting a diphosphine compound represented by the following general formula (2) 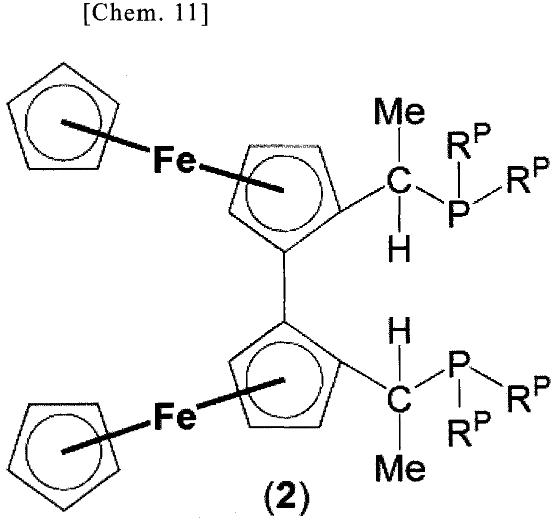 [Wherein,
   a solid line represents a single bond;
   H represents a hydrogen atom, C represents a carbon atom, and P represents a phosphorus atom;
   Me represents a methyl group;
   Fe represents a divalent iron ion, a pentagon containing a circle represents a cyclopentadienyl anion, and a bold line represents six-electron donation of the cyclopentadienyl anion to Fe;
   R^{P} represents a group selected from the group consisting of an alkyl group, a cycloalkyl group, a heteroaryl group, and an aryl group which may have a substituent.]
   with a ruthenium-alkylbenzene-carboxylate complex represented by the following general formula (3). 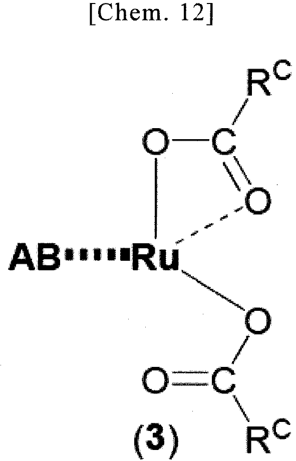
   [Wherein,
   a solid line represents a single bond, a double line represents a double bond, and a dashed line represents a coordinate bond;
   C represents a carbon atom, and O represents an oxygen atom;
   Ru represents a divalent ruthenium ion, AB represents an alkylbenzene, and a bold dashed line represents six-electron donation of the alkylbenzene to Ru;
   R^{C} represents a group selected from the group consisting of an alkyl group, a halogenoalkyl group, a cycloalkyl group, and an aryl group.]
[6] The method according to the above [5], wherein the diphosphine compound represented by the general formula (2) is an optically active form.
[7] The method according to the above [5] or [6], wherein R^{P} in the general formula (2) is an aryl group which may have a substituent.
[8] The method according to any one of the above [5] to [7], wherein R^{C} in the general formula (3) is selected from the group consisting of an alkyl group and a cycloalkyl group.
[9] The method according to any one of the above [5] to [8], wherein AB in the general formula (3) is selected from the group consisting of benzene, 1,3,5-trimethylbenzene, 1-methyl-4-isopropylbenzene, and hexamethylbenzene.
[10] A method for producing an optically active cyclic compound by the asymmetric hydrogenation of a compound selected from the group consisting of aromatic compounds and heteroaromatic compounds by using the ruthenium-diphosphine-carboxylate complex according to any one of the above [2] to [4] as a catalyst.
[11] The method according to the above [10], wherein the compound selected from the group consisting of aromatic compounds and heteroaromatic compounds is selected from the group consisting of indoles, oxazoles, imidazoles, and naphthalenes.

### Advantageous Effects of Invention

The present invention provides a ruthenium-diphosphine-carboxylate complex represented by the above general formula (1), namely Ru(O₂CR^{C})₂(R^{P}-trap) (hereinafter referred to as a ruthenium complex (1) of the present invention). The ruthenium complex (1) of the present invention can be easily produced by reacting a diphosphine compound represented by the above general formula (2) with a ruthenium-alkylbenzene-carboxylate complex represented by the above general formula (3) in a non-halogenated solvent that is readily available for industrial use. In addition, a preferred form of the ruthenium complex (1) of the present invention has excellent crystallinity, facilitating isolation, purification and long-term storage. Furthermore, this preferred form also exhibits excellent catalytic activity, asymmetric induction ability, and substrate generality without the addition of a base. Therefore, the present invention can contribute to improving the efficiency and facilitating the practical application of various organic synthesis reactions, including the asymmetric hydrogenation of aromatics which is useful for producing optically active cyclic compounds.

### Brief Description of Drawings

[Fig. 1] Fig. 1 shows the results of single-crystal X-ray structural analysis of Ru(O₂CMe)₂((S_{C},S_{C},R_{P},R_{P})-Ph-trap) produced in Example 1 below.
[Fig. 2] Fig. 2 shows the results of single-crystal X-ray structural analysis of Ru(O₂C^{t}Bu)₂((S_{C},S_{C},R_{P},R_{P})-Ph-trap) produced in Example 2 below.
[Fig. 3] Fig. 3 shows the results of single-crystal X-ray structural analysis of Ru(O₂C^{t}Bu)₂((R_{C},R_{C},S_{P},S_{P})-Ph-trap) produced in Example 3 below.
[Fig. 4] Fig. 4 shows the results of single-crystal X-ray structural analysis of Ru(O₂CAd)₂((S_{C},S_{C},R_{P},R_{P})-Ph-trap) produced in Example 4 below.
[Fig. 5] Fig. 5 shows the results of single-crystal X-ray structural analysis of Ru(O₂CAd)₂((R_{C},R_{C},S_{P},S_{P})-Ph-trap) produced in Example 5 below.
[Fig. 6] Fig. 6 shows the results of single-crystal X-ray structural analysis of Ru(O₂CAd)₂((R_{C},R_{C},S_{P},S_{P})-Ph-trap) ·H₂O produced in Example 5 below.
[Fig. 7] Fig. 7 shows the results of single-crystal X-ray structural analysis of (S)-N-Boc-2-phenylindoline produced in Example 9 below.
[Fig. 8] Fig. 8 shows the results of single-crystal X-ray structural analysis of (R)-N-Boc-2-phenylindoline produced in Example 14 below.
[Fig. 9] Fig. 9 shows the results of single-crystal X-ray structural analysis of (S)-N-Boc-3-phenylindoline produced in Example 17 below.

### Description of Embodiments

Hereinafter, the ruthenium complex (1) of the present invention is described in detail. In the above general formula (1), a solid line represents a single bond, a double line represents a double bond, and a dashed line represents a coordinate bond; H represents a hydrogen atom, C represents a carbon atom, O represents an oxygen atom, and P represents a phosphorus atom; Me represents a methyl group; Fe represents a divalent iron ion, a pentagon containing a circle represents a cyclopentadienyl anion, and a bold line represents six-electron donation of the cyclopentadienyl anion to Fe; R^{P} represents a group selected from the group consisting of an alkyl group, a cycloalkyl group, a heteroaryl group, and an aryl group which may have a substituent, and preferably represents an aryl group which may have a substituent; Ru represents a divalent ruthenium ion; R^{C} represents a group selected from the group consisting of an alkyl group, a halogenoalkyl group, a cycloalkyl group, and an aryl group, and preferably represents an alkyl group or a cycloalkyl group. In the ruthenium complex (1) of the present invention, all four R^{P}s present on the molecule represent the same group as each other, and both of the two R^{C}s present on the molecule represent the same group as each other.

The alkyl group, the cycloalkyl group, the heteroaryl group, and the aryl group which may have a substituent in R^{P} are described in more detail.

The alkyl group in R^{P} may be linear or branched; examples thereof include an alkyl group having 1 to 12 carbon atoms, preferably an alkyl group having 1 to 8 carbon atoms, and more preferably an alkyl group having 1 to 4 carbon atoms; specific examples thereof include a methyl group, an ethyl group, an n-propyl group, an isopropyl group, an n-butyl group, a sec-butyl group, an isobutyl group, and a tert-butyl group.

The cycloalkyl group in R^{P} may be monocyclic or polycyclic; examples thereof include a cycloalkyl group having 3 to 20 carbon atoms, preferably a cycloalkyl group having 3 to 15 carbon atoms, and more preferably a cycloalkyl group having 3 to 10 carbon atoms; specific examples thereof include a cyclopropyl group, a cyclobutyl group, a cyclopentyl group, a cyclohexyl group, a 1-adamantyl group, and a 2-adamantyl group.

Examples of the heteroaryl group in R^{P} include a heteroaryl group derived from a 5-membered aromatic heterocycle containing an oxygen atom or a sulfur atom; specific examples thereof include a 2-furyl group, a 3-furyl group, a 2-thienyl group, and a 3-thienyl group.

Examples of the aryl group in R^{P} include an aryl group having 6 to 18 carbon atoms, preferably an aryl group having 6 to 14 carbon atoms, and more preferably an aryl group having 6 to 10 carbon atoms; specific examples thereof include a phenyl group, a 1-naphthyl group, and a 2-naphtyl group; preferred specific examples thereof include a phenyl group. In addition, the aryl group may have a substituent.

Examples of the substituent that the aryl group in R^{P} may have include an alkyl group, a halogenoalkyl group, an aryl group, an alkoxy group, a dialkylamino group, and a halogeno group.

Examples of the alkyl group in these substituents include an alkyl group similar to those detailed in the description of R^{P}; specific examples thereof include a methyl group and a tert-butyl group; preferred specific examples thereof include a methyl group.

Examples of the halogenoalkyl group in these substituents include a halogenoalkyl group formed by substituting at least one hydrogen atom on the alkyl group described above with a halogen atom; specific examples thereof include a trifluoromethyl group.

Examples of the aryl group in these substituents include an aryl group similar to those detailed in the description of R^{P}; specific examples thereof include a phenyl group.

The alkoxy group in these substituents may be linear or branched; examples thereof include an alkoxy group having 1 to 12 carbon atoms, preferably an alkoxy group having 1 to 8 carbon atoms, and more preferably an alkoxy group having 1 to 4 carbon atoms; specific examples thereof include a methoxy group, an ethoxy group, an n-propoxy group, an isopropoxy group, an n-butoxy group, a sec-butoxy group, an isobutoxy group, and a tert-butoxy group.

Examples of the dialkylamino group in these substituents include a dialkylamino group formed by substituting two hydrogen atoms on an amino group with the alkyl group described above; specific examples thereof include an N,N-dimethylamino group.

Specific examples of the halogeno group in these substituents include a fluoro group, a chloro group, a bromo group, and an iodo group.

The alkyl group, the halogenoalkyl group, the cycloalkyl group, and the aryl group in R^{C} are described in more detail.

The alkyl group in R^{C} may be linear or branched; examples thereof include an alkyl group having 1 to 12 carbon atoms, preferably an alkyl group having 1 to 8 carbon atoms, and more preferably an alkyl group having 1 to 4 carbon atoms; specific examples thereof include a methyl group, an ethyl group, an n-propyl group, an isopropyl group, an n-butyl group, a sec-butyl group, an isobutyl group, and a tert-butyl group; preferred specific examples thereof include a methyl group and a tert-butyl group.

Examples of the halogenoalkyl group in R^{C} include a halogenoalkyl group formed by substituting at least one hydrogen atom on the alkyl group described above with a halogen atom; specific examples thereof include a trifluoromethyl group.

The cycloalkyl group in R^{C} may be monocyclic or polycyclic; examples thereof include a cycloalkyl group having 3 to 20 carbon atoms, preferably a cycloalkyl group having 3 to 15 carbon atoms, and more preferably a cycloalkyl group having 3 to 10 carbon atoms; specific examples thereof include a cyclopropyl group, a cyclobutyl group, a cyclopentyl group, a cyclohexyl group, a 1-adamantyl group, and a 2-adamantyl group; preferred specific examples thereof include a 1-adamantyl group.

Examples of the aryl group in R^{C} include an aryl group having 6 to 18 carbon atoms, preferably an aryl group having 6 to 14 carbon atoms, and more preferably an aryl group having 6 to 10 carbon atoms; specific examples thereof include a phenyl group, a 1-naphthyl group, and a 2-naphtyl group.

The ruthenium complex (1) of the present invention has two carbon-centered chiral structures (hereinafter, the absolute configurations of these carbon-centered chiral structures are represented by Rc or Sc), two planar chiral structures (hereinafter, the absolute configurations of these planar chiral structures are represented by R_{P} or S_{P}), and a secondarily induced ruthenium-centered chiral structure (hereinafter, the absolute configurations of the ruthenium-centered chiral structure are represented by Δ or A). Accordingly, the ruthenium complex (1) of the present invention may be either a mixture of stereoisomers arising from these chiral structures or a single stereoisomer; however, from the viewpoint of application to the asymmetric hydrogenation of aromatics in particular, it is preferably a single stereoisomer, namely, an optically active form.

Specific examples of preferred form of the optically active ruthenium complex (1) of the present invention include Ru(O₂CR^{C})₂((S_{C},S_{C},R_{P},R_{P})-R^{P}-trap) ((S_{C},S_{C},R_{P},R_{P})-1) and Ru(O₂CR^{C})₂ ((R_{C},R_{C},S_{P},S_{P})-R^{P}-trap) ((R_{C},R_{C},S_{P},S_{P})-1) due to their structural requirements, whose stereochemical structures are shown in Formula 10 below (in accordance with convention, the secondarily induced ruthenium-centered chiral structure, the carbon atoms (C), and the hydrogen atoms (H) are omitted in the drawings of stereochemical structures herein). 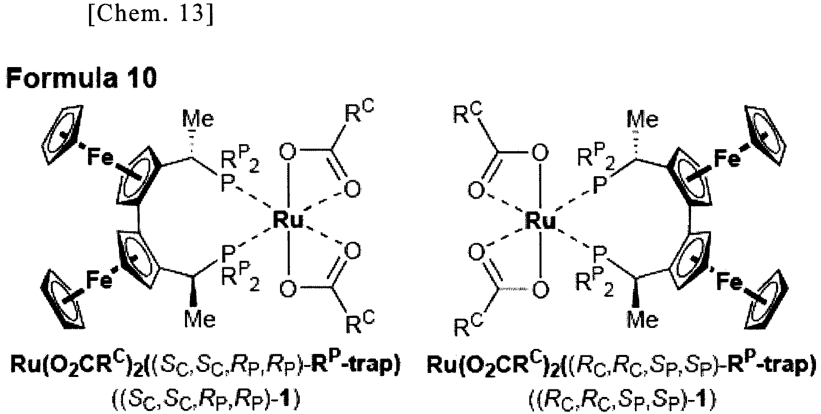 [In the formula, a solid wedge line represents a carbon-carbon bond projecting toward the viewer, and a dashed wedge line represents a carbon-carbon bond extending away from the viewer.]

Particularly preferred specific examples of the ruthenium complex (1) of the invention include Ru(O₂CMe)₂((S_{C},S_{C},R_{P},R_{P})-Ph-trap), Ru(O₂CMe)₂((R_{C},R_{C},S_{P},S_{P})-Ph-trap), Ru(O₂C^{t}Bu)₂((S_{C},S_{C},R_{P},R_{P})-Ph-trap), Ru(O₂C^{t}Bu)₂((R_{C},R_{C},S_{P},S_{P})-Ph-trap), Ru(O₂CAd)₂((S_{C},S_{C},R_{P},R_{P})-Ph-trap), and Ru(O₂CAd)₂((R_{C},R_{C},S_{P},S_{P})-Ph-trap), whose stereochemical structures are shown in Formula 11 below; Ru(O₂CMe)₂((S_{C},S_{C},R_{P},R_{P})-Tol-trap), Ru(O₂CMe)₂((R_{C},R_{C},S_{P},S_{P})-Tol-trap), Ru(O₂C^{t}Bu)₂((S_{C},S_{C},R_{P},R_{P})-Tol-trap), Ru(O₂C^{t}Bu)₂((R_{C},R_{C},S_{P},S_{P})-Tol-trap), Ru(O₂CAd)₂((S_{C},S_{C},R_{P},R_{P})-Tol-trap), and Ru(O₂CAd)₂((R_{C},R_{C},S_{P},S_{P})-Tol-trap), whose stereochemical structures are shown in Formula 12 below. 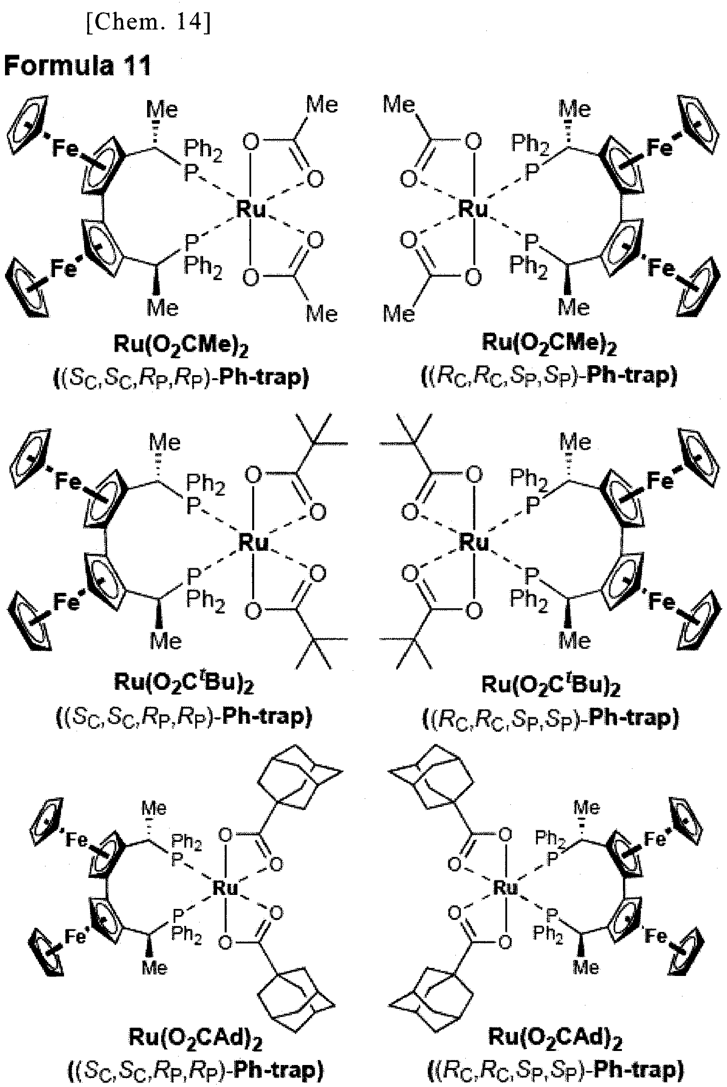 [In the formula, Ph represents a phenyl group, and Me represents a methyl group.] 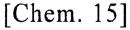 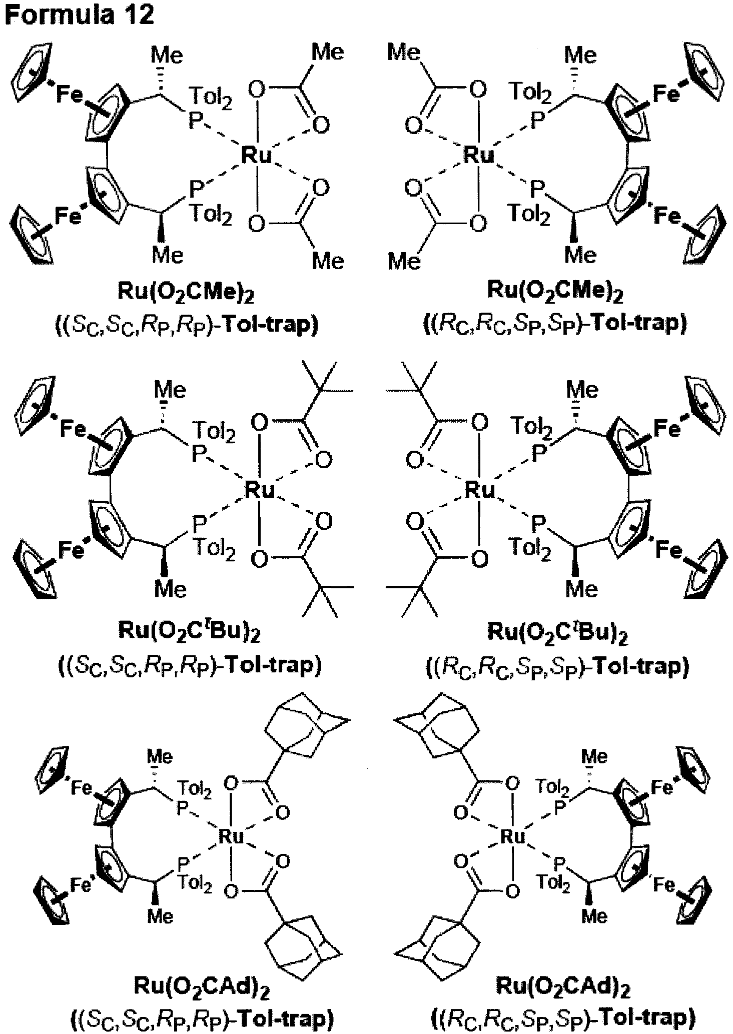 [In the formula, Tol represents a 4-methylphenyl group, and Me represents a methyl group.]

Hereinafter, the method for producing the ruthenium complex (1) of the present invention (hereinafter referred to as the production method of the present invention) is described in detail. The ruthenium complex (1) of the present invention can be readily produced by reacting a diphosphine compound represented by the above general formula (2) (hereinafter referred to as R^{P}-TRAP (2)) with a ruthenium-alkylbenzene-carboxylate complex represented by the above general formula (3), namely Ru(O₂CR^{C})₂(AB) (3) (hereinafter referred to as a ruthenium source (3)), while dissociating an alkylbenzene, referred to as AB, as shown in Formula 13 below.

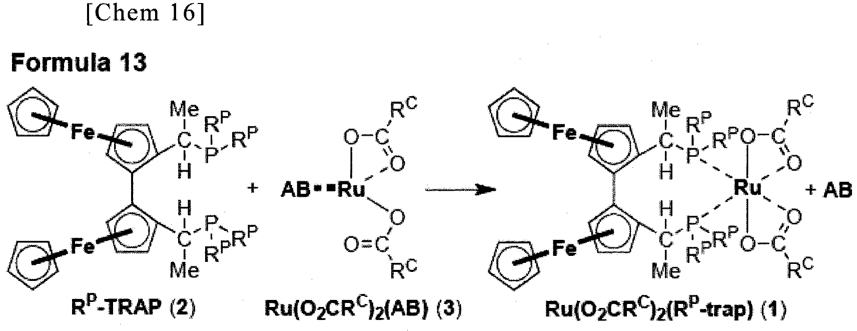

Hereinafter, R^{P}-TRAP (2) in the production method of the present invention is described in detail. In the above general formula (2), the solid line, H, C, P, Me, Fe, the pentagon containing a circle, the bold line, and R^{P} are the same as those defined and detailed in the description of the above general formula (1). In R^{P}-TRAP (2), all four R^{P}s present on the molecule represent the same group as each other.

R^{P}-TRAP (2) has two carbon-centered chiral structures and two planar chiral structures, and therefore may be either a mixture of stereoisomers arising from these chiral structures or a single stereoisomer; however, from the viewpoint of application to the asymmetric hydrogenation of aromatics in particular, it is preferably a single stereoisomer, namely an optically active form, as with the ruthenium complex (1) of the present invention. Specific examples of preferred form of the optically active R^{P}-TRAP (2) include (S_{C},S_{C},R_{P},R_{P})-R^{P}-TRAP ((S_{C},S_{C},R_{P},R_{P})-2) and (R_{C},R_{C},S_{P},S_{P})-R^{P}-TRAP ((R_{C},R_{C},S_{P},S_{P})-2) due to their structural requirements, whose stereochemical structures are shown in Formula 14 below.

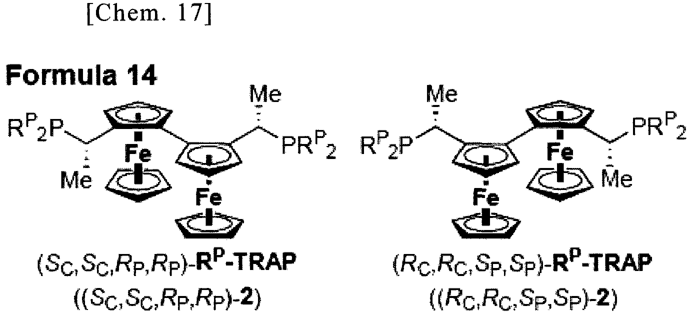

Particularly preferred specific examples of R^{P}-TRAP (2) in the production method of the present invention include (S_{C},S_{C},R_{P},R_{P})-Ph-TRAP, (R_{C},R_{C},S_{P},S_{P})-Ph-TRAP, (S_{C},S_{C},R_{P},R_{P})-Tol-TRAP, and (R_{C},R_{C},S_{P},S_{P})-Tol-TRAP, whose stereochemical structures are shown in Formula 15 below. 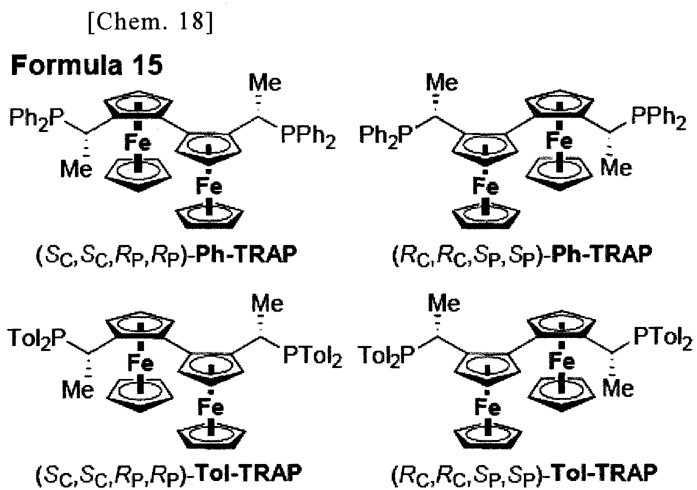 [In the formula, Ph represents a phenyl group, and Tol represents a 4-methylphenyl group.]

The synthesis method of R^{P}-TRAP (2) is not particularly limited; however, from a practical standpoint, the methods described in International Publication No. WO2024/203802 and International Publication No. WO2024/203803 are preferred, and their outline is shown in Formula 16 below. Specifically, R^{P}-TRAP (2) can be readily synthesized in multiple steps with good reproducibility, starting from commercially available N,N-dimethyl-1-ferrocenylethylamine (commonly known as Ugi's Amine), through lithiation and halogenation (Step 1), phosphination (Step 2), reaction with a borane source (Step 3), reaction with a magnesium source (Step 4), reaction with an oxidizing agent (Step 5), and deprotection reaction (Step 6). In addition, (S_{C},S_{C},R_{P},R_{P})-R^{P}-TRAP ((S_{C},S_{C},R_{P},R_{P})-2) and (R_{C},R_{C},S_{P},S_{P})-R^{P}-TRAP ((R_{C},R_{C},S_{P},S_{P})-2) described above, which are the preferred forms of optically active R^{P}-TRAP (2), can be selectively synthesized by applying the optically active form of Ugi's Amine to these synthesis methods. Furthermore, R^{P}-TRAP (2) prepared by these synthesis methods may form a stable solvate with the solvent used in the deprotection reaction (Step 6) described above; preferred specific examples of such solvents include n-propyl alcohol and n-butyl alcohol. 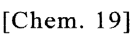 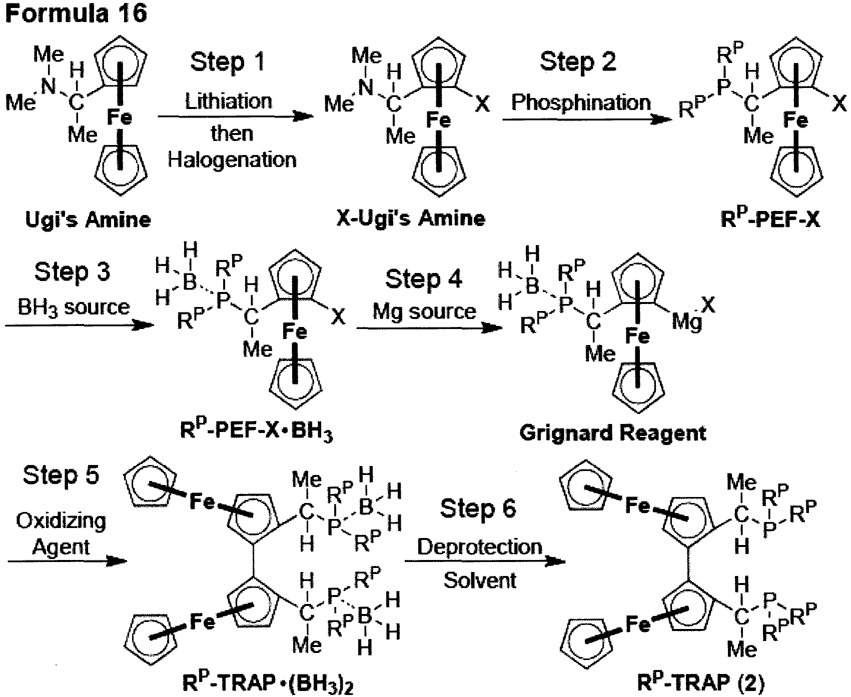 [In the formula, B represents a boron atom, Mg represents a magnesium atom, N represents a nitrogen atom, and X represents a halogen atom.]

Hereinafter, the ruthenium source (3) in the production method of the present invention is described in detail. In the above general formula (3), the solid line, the double line, the dashed line, C, O, Ru, and R^{C} are the same as those defined and detailed in the description of the above general formula (1). AB represents an alkylbenzene, and a bold-dashed line represents six-electron donation of the alkylbenzene to Ru. In the ruthenium source (3), both of the two R^{C}s present on the molecule represent the same group as each other.

Hereinafter, the alkylbenzene as AB is described in detail. Examples of the alkylbenzene include benzene (C₆H₆) and a compound in which at least one hydrogen atom on benzene is substituted with an alkyl group; examples thereof include an alkylbenzene having 6 to 24 carbon atoms, preferably an alkylbenzene having 6 to 18 carbon atoms, and more preferably an alkylbenzene having 6 to 12 carbon atoms; specific examples thereof include benzene, 1,3,5-trimethylbenzene (mesitylene), 1-methyl-4-isopropylbenzene (p-cymene), and hexamethylbenzene; preferred specific examples thereof include 1-methyl-4-isopropylbenzene (p-cymene).

Particularly preferred specific examples of the ruthenium source (3) in the production method of the present invention include Ru(O₂CMe)₂(p-cymene), Ru(O₂C^{t}Bu)₂(p-cymene), and Ru(O₂CAd)₂(p-cymene), whose structural formulae are shown in Formula 17 below. All of these ruthenium sources (3) can be readily synthesized according to the known methods. 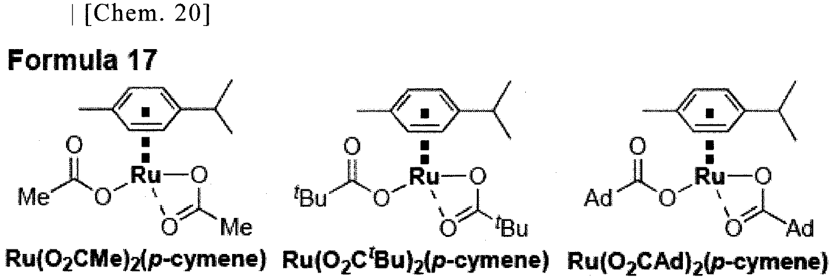 [In the formula, Me represents a methyl group, ^{t}Bu represents a tert-butyl group, and Ad represents a 1-adamantyl group.]

Hereinafter, an embodiment of the production method of the present invention, specifically the reaction conditions between R^{P}-TRAP (2) and the ruthenium source (3), is described in more detail. The amount of R^{P}-TRAP (2) used in the reaction is not particularly limited; however, from the standpoint of atom efficiency, it is appropriately selected from the range of usually 0.5 to 1.5 equivalents, preferably 0.8 to 1.2 equivalents, and more preferably 0.9 to 1.1 equivalents relative to the amount of the ruthenium source (3) used.

In principle, the reaction of R^{P}-TRAP (2) with the ruthenium source (3) can be conducted under solvent-free conditions; however, such conditions require special equipment such as a ball mill or a kneader. Therefore, the reaction is preferably carried out in the presence of a solvent for practical purposes. Preferred examples of the solvent include a solvent that does not contribute to the decomposition of the ruthenium complex (1) of the present invention; preferred specific examples thereof include: aliphatic hydrocarbons such as n-pentane, n-hexane, n-heptane, n-octane, n-decane, cyclohexane, and decalin; aromatic hydrocarbons such as benzene, toluene, xylenes, mesitylene, p-cymene, and 1,4-diisopropylbenzene; ethers such as diethyl ether, diisopropyl ether, tert-butyl methyl ether, cyclopentyl methyl ether, 1,2-dimethoxyethane, 1,2-diethoxyethane, tetrahydrofuran, 2-methyltetrahydrofuran, 4-methyltetrahydropyran, and 1,4-dioxane; ketones such as acetone, methyl ethyl ketone, and methyl iso-butyl ketone; and water; more preferred specific examples thereof include aromatic hydrocarbons such as benzene, toluene, xylenes, mesitylene, p-cymene, and 1,4-diisopropylbenzene; particularly preferred specific examples thereof include toluene, which is inexpensive, readily handled, and exhibits excellent ability to dissolve the substrates. These solvents may be used alone or in combination of two or more. The amount of the solvent used is not particularly limited; however, it is appropriately selected from the range of usually 1 to 200 times by volume, preferably 1.5 to 100 times by volume, and more preferably 2 to 50 times by volume relative to the weight of R^{P}-TRAP (2) used.

The reaction of R^{P}-TRAP (2) with the ruthenium source (3) is preferably carried out under an inert gas atmosphere in order to suppress decomposition of substrates or reaction intermediates; specific examples of the inert gas include argon gas and nitrogen gas; preferred specific examples thereof include nitrogen gas. The reaction temperature is appropriately selected from the range of usually -20°C to 160°C, preferably 0°C to 140°C, and more preferably 20°C to 120°C. The reaction time depends on the chemical structure of R^{P}-TRAP (2) and the ruthenium source (3), the amount of R^{P}-TRAP (2) used, the reaction solvent, and the reaction temperature; however, it is appropriately selected from the range of usually 5 minutes to 24 hours, preferably 10 minutes to 12 hours, and more preferably 20 minutes to 6 hours.

The reaction solution obtained from R^{P}-TRAP (2), the ruthenium source (3), and the solvent described above may be subjected to post-treatment as necessary, and the ruthenium complex (1) of the present invention may be isolated from the reaction solution and further purified. Specific examples of the post-treatment methods include filtration, concentration, and solvent-replacement of the reaction solution. Specific examples of the isolation methods include: concentration to dryness or crystallization of the reaction solution; filtration, washing, and drying of crude crystals. Specific examples of the purification methods include: dissolution, decolorization with an adsorbent, and recrystallization of the crude crystals; filtration, washing, and drying of purified crystals. These methods may be carried out alone or in combination of two or more. Furthermore, the ruthenium complex (1) of the present invention may contain the solvents used in the reaction, post-treatment, isolation or purification; or may contain the alkylbenzene dissociating during the reaction. Preferred specific examples of such solvents and an alkylbenzene include toluene, n-heptane, acetone, water, and p-cymene.

When using the ruthenium complex (1) of the present invention as a catalyst, the reaction solution described above may be used as is, or it may be used after performing the post-treatment, isolation, or purification described above as necessary. On the other hand, the preferred form of the ruthenium complex (1) of the present invention exhibits excellent crystallinity and stability, facilitating long-term storage. Therefore, from the standpoint of utilizing these properties, it is preferable to use it as a catalyst after isolation. The ruthenium complex (1) of the present invention can be used as a catalyst alone or in combination of two or more; however, from a practical standpoint, it is preferable to use it as a catalyst alone.

The ruthenium complex (1) of the present invention produced in this method can be suitably used as a catalyst in various organic synthesis reactions. These organic synthesis reactions are not particularly limited; however, specific examples thereof include oxidation reactions, reduction reactions, hydrogenation reactions, dehydrogenation reactions, hydrogen transfer reactions, addition reactions, conjugate addition reactions, pericyclic reactions, functional group conversion reactions, isomerization reactions, rearrangement reactions, polymerization reactions, bond-formation reactions, and bond-cleavage reactions. All of these organic synthesis reactions may be asymmetric form; preferred specific examples thereof include the asymmetric hydrogenation of aromatic compounds and heteroaromatic compounds, namely, the asymmetric hydrogenation of aromatics.

Particularly preferred specific examples of the asymmetric hydrogenation of aromatics include the asymmetric hydrogenation of indoles, oxazoles, imidazoles, and naphthalenes under neutral conditions, which enable the efficient production of industrially useful optically active cyclic compounds.

### Examples

Hereinafter, the method for producing the ruthenium complex (1) of the present invention and the asymmetric hydrogenation of aromatics catalyzed by this complex will be described in detail with reference to specific examples and comparative examples; however, the present invention is not limited in any way by these descriptions. Unless otherwise noted, substrates, reagents, and solvents were charged or added under a nitrogen stream; reactions were carried out under a nitrogen atmosphere; post-treatment, isolation, and purification were performed in air. The apparatus and measurement/analysis conditions used for measuring physical properties in the examples are listed below:
1) Proton Nuclear Magnetic Resonance Spectroscopy (¹H NMR): 400MR DD2 Type Apparatus (Resonance Frequency: 400 MHz; manufactured by Agilent Technologies, Inc.)
2) Carbon 13 Nuclear Magnetic Resonance Spectroscopy (¹³C NMR): 400MR DD2 Type Apparatus (Resonance Frequency: 100 MHz; manufactured by Agilent Technologies, Inc.)
3) Phosphorus 31 Nuclear Magnetic Resonance Spectroscopy (³¹P NMR): 400MR DD2 Type Apparatus (Resonance Frequency: 161 MHz; manufactured by Agilent Technologies, Inc.)
4) High Performance Liquid Chromatography (HPLC): GL-7400 Type Apparatus (manufactured by GL Sciences, Inc.)
5) Single-Crystal X-ray Structural Analysis: XtaLAB Synergy-S Type Apparatus (manufactured by Rigaku Oxford Diffraction)

[Measurement Conditions] X-ray Source: CuKα radiation; Device Control Program: CrysAlis^{PRO}.
[Analysis Conditions] Structural Analysis Software: Olex² 1.3-ac4; Structural Analysis Program: SHELXS/SHELXL-2018/3; Drawing Software: Mercury 4.3.0.

### [Example 1] Production of Ru(O₂CMe)₂((S_{C},S_{C},R_{P},R_{P})-Ph-trap)

### (Formula 18)

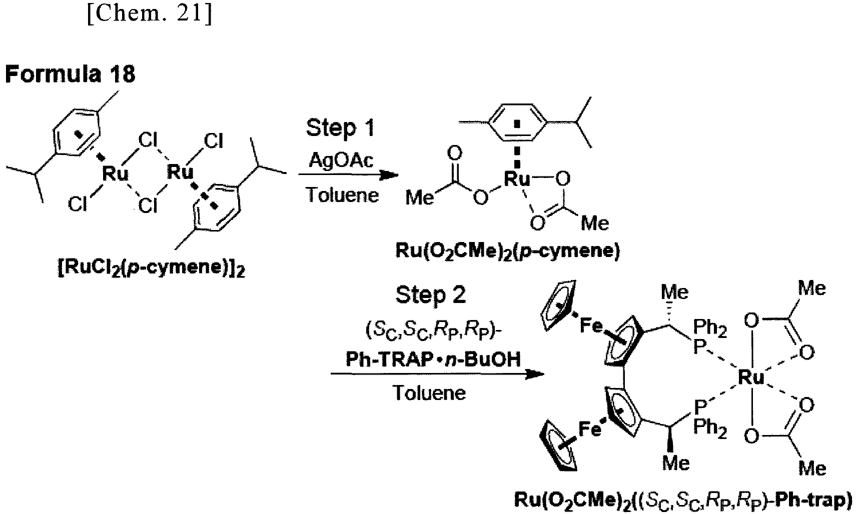

### Step1: Preparation of Ru(O₂CMe)₂(p-cymene) solution

[Setup and Reaction] A 50 mL, two-necked, round-bottomed flask was successively charged with dichloro(p-cymene)ruthenium dimer ([RuCl₂(p-cymene)]₂; 716 mg, 1.17 mmol, 1.0 equivalent) and silver acetate (AgOAc; 781 mg, 4.68 mmol, 4.0 equivalents), equipped with a magnetic stirring bar and a three-way stopcock, and then evacuated and backfilled with nitrogen. After adding dehydrated toluene (15 mL), this flask was covered with aluminum foil to protect it from light. The contents of the covered flask were stirred for 2 hours at room temperature.

[Post-Treatment] The resulting orange suspension was filtered using diatomaceous earth under a nitrogen stream, and the filtered residue was washed with dehydrated toluene (15 mL) to afford the desired toluene solution (approximately 30 mL) of Ru(O₂CMe)₂(p-cymene) (<2.34 mmol) as a reddish-orange liquid. This complex could also be isolated as hygroscopic reddish-brown crystals by concentrating its toluene solution and then adding n-heptane for crystallization. The results of NMR analysis of this complex were as follows;

¹H NMR (400 MHz, CDCl₃): δ = 5.78 (d, J = 5.6 Hz, 2H), 5.57 (d, J = 5.6 Hz, 2H), 2.87 (sept, J = 7.2 Hz, 1H), 2.26 (s, 3H), 1.94 (s, 6H), 1.36 (d, J = 7.2 Hz, 6H).
¹³C NMR (100 MHz, CDCl₃): δ = 184.37, 97.98, 92.59, 78.84, 77.68, 31.38, 23.77, 22.46, 18.50.

### Step 2: Production of Ru(O₂CMe)₂((S_{C},S_{C},R_{P},R_{P})-Ph-trap)

[Setup and Reaction] A 100 mL, four-necked, round-bottomed flask was charged with (S_{C},S_{C},R_{P},R_{P})-Ph-TRAP·n-BuOH (purity: 92.9% by weight, 2.0 g, 2.34 mmol, 1.0 equivalent) synthesized according to the method described in International Publication No. WO2024/203803, equipped with a magnetic stirring bar, thermometer, dropping funnel, Claisen distillation apparatus, Dimroth condenser, and a three-way stopcock, and then evacuated and backfilled with nitrogen. Dehydrated toluene (40 mL) was added to this flask, and the resulting orange solution was stirred at 60°C while gradually reducing the pressure inside the apparatus to 60 Torr. After recovering approximately 30 mL of solvent, the inside of the apparatus was filled with nitrogen gas. The toluene solution (approximately 30 mL) of Ru(O₂CMe)₂(p-cymene) prepared in Step 1 (<2.34 mmol, 1.0 equivalent) was charged into the dropping funnel, and then added dropwise to the reaction solution at room temperature. The resulting mixture was stirred for 2 hours while being heated using an oil bath set at 70°C.

[Post-Treatment, Isolation, and Purification] The dark-brown reaction solution was stirred at 70°C while gradually reducing the pressure inside the apparatus to 60 Torr. After recovering approximately 30 mL of solvent, the inside of the apparatus was filled with nitrogen gas. Dehydrated n-heptane (40 mL) was added to the concentrated solution while being stirred at 60°C. The resulting brown slurry was cooled to 0°C using an ice-water bath, and then filtered by suction using a Kiriyama funnel under a nitrogen stream. The filtered crystals were washed twice with a mixture of dehydrated n-heptane (8 mL) and dehydrated toluene (1 mL) cooled to 0°C, and then dried by heating at 60°C under reduced pressure of 1 Torr for 1 hour, producing 2.34 g of the desired Ru(O₂CMe)₂((S_{C},S_{C},R_{P},R_{P})-Ph-trap) as an air-stable light-brown powder. Purity: 92.1% by weight (main impurities were 7.5% by weight of toluene and 0.4% by weight of n-heptane); Overall Yield: 90.9%. Single crystals of this complex were successfully prepared by solution diffusion using toluene as a good solvent and n-heptane as a poor solvent.

¹H NMR (400 MHz, C₆D₆): δ = 8.06 (bs, 4H), 7.47 (bs, 4H), 7.24-6.90 (m, 12H [Target] + 4.50H [Toluene]), 4.69 (bs, 2H), 4.22 (bs, 2H), 4.17 (t, J = 2.4 Hz, 2H), 4.04 (bs, 2H), 4.01 (s, 10H), 2.10 (s, 2.70H [Toluene]), 1.67 (s, 6H), 1.25-1.00 (bs, 6H [Target] + 0.40H [n-Heptane]), 0.87 (t, J = 6.8 Hz, 0.24H [n-Heptane]).
³¹P NMR (161 MHz, C₆D₆): δ = 74.07 (s, 2P).

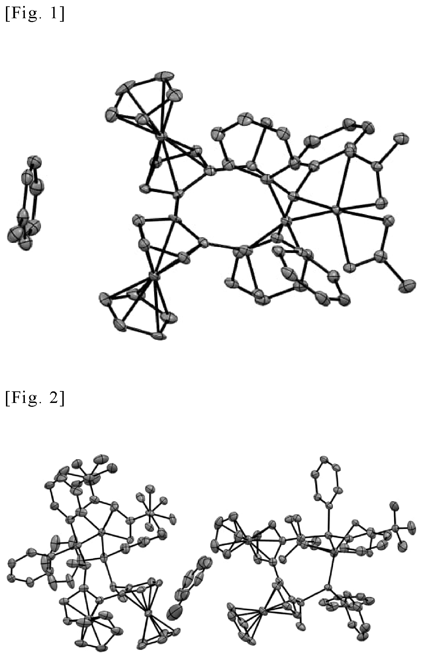

Fig. 1 shows the results of single-crystal X-ray structural analysis (thermal ellipsoid plot; 50% probability level) of Ru(O₂CMe)₂((S_{C},S_{C},R_{P},R_{P})-Ph-trap) produced in Example 1. Note that the depiction of hydrogen atoms in the figure is omitted for clarity of the structure of this complex. The results clearly confirmed that: 1) this complex crystallizes while solvating one molecule of toluene; 2) this complex exhibits C₂ symmetry in its crystalline state; 3) its secondarily induced ruthenium-centered chiral structure is Δ; 4) surprisingly, Ph-TRAP in this complex functions as a cis-chelating chiral diphosphine ligand (bite angle: 91.42°), rather than the conventionally known trans-chelating ligand (bite angle: approximately 180°). The key parameters ensuring the reliability of the results of analysis are summarized below; Chemical Formula: C₅₉H₅₈Fe₂O₄P₂Ru; Crystal System: Orthorhombic; Space Group: P2₁2₁2₁ (#19); Lattice Constants: a = 11.11914(15) Å, b = 17.4009(2) Å, c = 25.5514(3) Å, α = β = γ = 90°; Reliability Factor (R₁): 0.0407; Weighted Reliability Factor (wR₂): 0.0814; Goodness of Fit (GOF): 1.019; Flack Parameter: -0.025(4).

### [Example 2] Production of Ru(O₂C^{t}Bu)₂((S_{C},S_{C},R_{P},R_{P})-Ph-trap)

### (Formula 19)

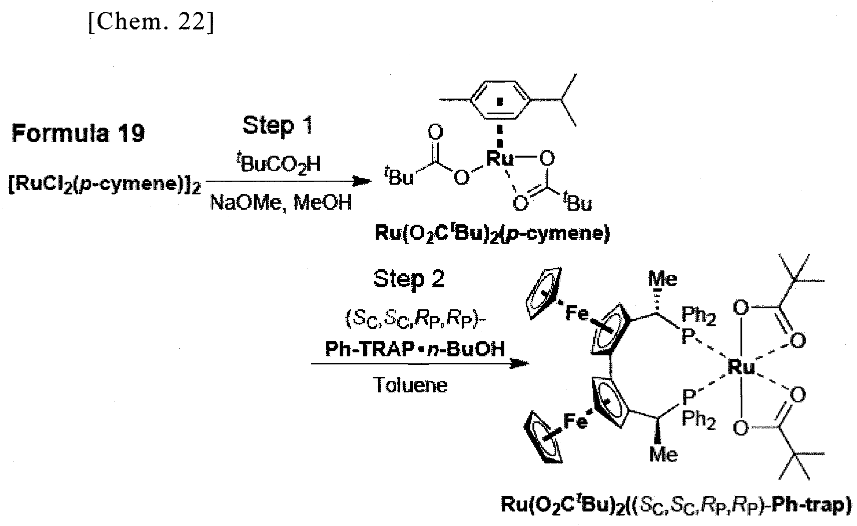

### Step 1: Synthesis of Ru(O₂C^{t}Bu)₂(p-cymene)

[Setup and Reaction] A 200 mL, four-necked, round-bottomed flask was sequentially charged with pivalic acid (^{t}BuCO₂H; 3.67 g, 35.9 mmol, 4.4 equivalents) and [RuCl₂(p-cymene)]₂ (5.0 g, 8.16 mmol, 1.0 equivalent), equipped with a magnetic stirring bar, thermometer, Claisen distillation apparatus, Dimroth condenser, and a three-way stopcock, and then evacuated and backfilled with nitrogen. Dehydrated methanol (MeOH; 40 mL) and a methanol solution of sodium methoxide (NaOMe) (purity: 28.0% by weight, 6.89 mL, 34.3 mmol, 4.2 equivalents) were sequentially added to this flask. The contents of the flask were stirred for 2 hours while being heated using an oil bath set at 60°C.

[Post-Treatment, Isolation, and Purification] Toluene (80 mL) was added to the reaction solution, and the resulting mixture was stirred at 60°C while gradually reducing the pressure inside the apparatus to 60 Torr. After recovering approximately 80 mL of solvent, the inside of the apparatus was filled with nitrogen gas. Toluene (120 mL) was added to the resulting reddish-orange slurry, and the mixture was filtered using diatomaceous earth under a nitrogen stream. The filtered residue was washed with toluene (40 mL), and the combined filtrate was transferred to a 500 mL, four-necked, round-bottomed flask equipped with a magnetic stirring bar, thermometer, Claisen distillation apparatus, and a three-way stopcock. The filtrate in the flask was stirred at 60°C while gradually reducing the pressure inside the apparatus to 50 Torr. After recovering approximately 130 mL of solvent, the inside of the apparatus was filled with nitrogen gas. To the concentrated filtrate was added n-heptane (90 mL), and the mixture was stirred at 60°C while gradually reducing the pressure inside the apparatus to 70 Torr. After recovering approximately 90 mL of solvent, the inside of the apparatus was filled with nitrogen gas. To the resulting orange slurry was added n-heptane (90 mL), and the mixture was cooled to 0°C using an ice-water bath, and then filtered by suction using a Kiriyama funnel. The filtered crystals were washed twice with n-heptane (10 mL) cooled to 0°C, and then dried by heating at 60°C under reduced pressure of 1 Torr for 1 hour, affording 6.79 g of the desired Ru(O₂C^{t}Bu)₂(p-cymene) as an air-stable yellowish-orange powder. Isolated Yield: 95.1%.

¹H NMR (400 MHz, CDCl₃): δ = 5.72 (d, J = 6.0 Hz, 2H), 5.50 (d, J = 6.0 Hz, 2H), 2.89 (sept, J = 7.2 Hz, 1H), 2.26 (s, 3H), 1.35 (d, J = 7.2 Hz, 6H), 1.08 (s, 18H).
¹³C NMR (100 MHz, CDCl₃): δ = 191.85, 98.59, 94.10, 78.21, 76.76, 39.86, 31.48, 27.60, 22.46, 18.47.

### Step 2: Production of Ru(O₂C^{t}Bu)₂((S_{C},S_{C},R_{P},R_{P})-Ph-trap)

[Setup and Reaction] A 50 mL, four-necked, round-bottomed flask was charged with (S_{C},S_{C},R_{P},R_{P})-Ph-TRAP·n-BuOH (purity: 92.9% by weight, 1.0 g, 1.17 mmol, 1.0 equivalent), equipped with a magnetic stirring bar, thermometer, Claisen distillation apparatus, Dimroth condenser, and a three-way stopcock, and then evacuated and backfilled with nitrogen. Dehydrated toluene (20 mL) was added to this flask, and the resulting orange solution was stirred at 60°C while gradually reducing the pressure inside the apparatus to 60 Torr. After recovering approximately 15 mL of solvent, the inside of the apparatus was filled with nitrogen gas. Ru(O₂C^{t}Bu)₂(p-cymene) synthesized in Step 1 (512 mg, 1.17 mmol, 1.0 equivalent) was quickly added to the reaction solution at room temperature. The resulting mixture was stirred for 2 hours while being heated using an oil bath set at 70°C.

[Post-Treatment, Isolation, and Purification] After allowing the resulting brown slurry to cool to room temperature, dehydrated n-heptane (10 mL) was added to the slurry, and the resulting mixture was filtered by suction using a Kiriyama funnel under a nitrogen stream. The filtered crystals were washed with a mixture of dehydrated n-heptane (8 mL) and dehydrated toluene (2 mL), and then dried by heating at 60°C under reduced pressure of 1 Torr for 1 hour, producing 1.16 g of the desired Ru(O₂C^{t}Bu)₂((S_{C},S_{C},R_{P},R_{P})-Ph-trap) as an air-stable orange powder. Purity: 96.8% by weight (main impurity was 3.4% by weight of toluene); Isolated Yield: 87.4%. Single crystals of this complex were successfully prepared by solution diffusion using toluene as a good solvent and n-heptane as a poor solvent.

¹H NMR (400 MHz, C₆D₆): δ = 7.62 (bs, 4H), 7.45-7.37 (bt, 4H), 7.14-6.93 (m, 12H [Target] + 1.97H [Toluene]), 4.46 (bs, 2H), 4.06 (s, 10H), 4.04 (t, J = 2.4 Hz, 2H), 3.80 (bs, 2H), 3.74 (bs, 2H), 2.10 (s, 1.18H [Toluene]), 1.42 (bs, 6H), 1.12 (s, 18H).
³¹P NMR (161 MHz, C₆D₆): δ = 72.13 (s, 2P).

Fig. 2 shows the results of single-crystal X-ray structural analysis (thermal ellipsoid plot; 50% probability level) of Ru(O₂C^{t}Bu)₂((S_{C},S_{C},R_{P},R_{P})-Ph-trap) produced in Example 2. Note that the depiction of hydrogen atoms in the figure is omitted for clarity of the structure of this complex. The results clearly confirmed that: 1) this complex crystallizes while solvating one molecule of toluene for every two molecules of the complex; 2) this complex exhibits C₁ symmetry in its crystalline state; 3) its secondarily induced ruthenium-centered chiral structure is Δ; 4) surprisingly, Ph-TRAP in this complex functions as a cis-chelating chiral diphosphine ligand (average bite angle: 107.43°), rather than the conventionally known trans-chelating ligand (bite angle: approximately 180°). The key parameters ensuring the reliability of the results of analysis are summarized below; Chemical Formula: C₁₂₃H₁₃₂Fe₄O₈P₄Ru₂; Crystal System: Monoclinic; Space Group: P1 2₁ 1 (#4); Lattice Constants: a = 11.32810(13) Å, b = 43.8943(4) Å, c = 11.49857(13) Å, α = γ = 90°, β = 111.2182(13)° ; R₁: 0.0450; wR₂: 0.1031; GOF: 1.034; Flack Parameter: -0.013(3).

### [Example 3] Production of Ru(O₂C^{t}Bu)₂((R_{C},R_{C},S_{P},S_{P})-Ph-trap)

### (Formula 20)

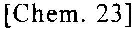

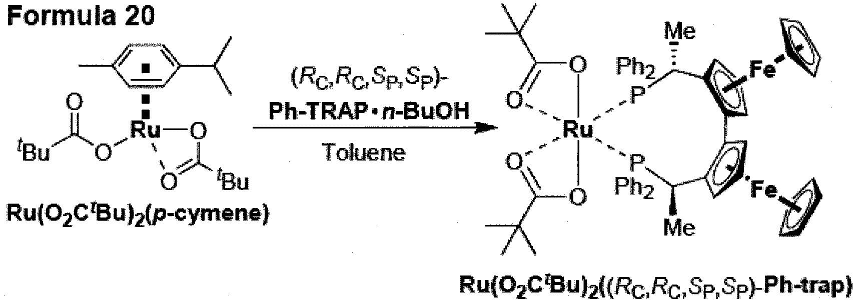

[Setup and Reaction] A 50 mL, four-necked, round-bottomed flask was charged with (R_{C},R_{C},S_{P},S_{P})-Ph-TRAP·n-BuOH (purity: 93.8% by weight, 1.0 g, 1.18 mmol, 1.0 equivalent) synthesized according to the method described in International Publication No. WO2024/203803, equipped with a magnetic stirring bar, thermometer, dropping funnel, Claisen distillation apparatus, Dimroth condenser, and a three-way stopcock, and then evacuated and backfilled with nitrogen. Dehydrated toluene (20 mL) was added to this flask, and the resulting orange solution was stirred at 60°C while gradually reducing the pressure inside the apparatus to 60 Torr. After recovering approximately 15 mL of solvent, the inside of the apparatus was filled with nitrogen gas. Ru(O₂C^{t}Bu)₂(p-cymene) synthesized in Step 1 of Example 2 (492 mg, 1.12 mmol, 1.0 equivalent) and dehydrated toluene (15 mL) were sequentially charged into the dropping funnel. The resulting suspension was added dropwise to the reaction solution at room temperature, followed by stirring for 2 hours while being heated using an oil bath set at 70°C.

[Post-Treatment, Isolation, and Purification] The dark-red reaction solution was stirred at 60°C while gradually reducing the pressure inside the apparatus to 60 Torr. After recovering approximately 15 mL of solvent, the inside of the apparatus was filled with nitrogen gas. Dehydrated n-heptane (15 mL) was added to the resulting brown slurry while being stirred at room temperature. The slurry was cooled to 0°C using an ice-water bath, and then filtered by suction using a Kiriyama funnel under a nitrogen stream. The filtered crystals were washed with a mixture of dehydrated n-heptane (10 mL) and dehydrated toluene (1 mL) cooled to 0°C, and then dried by heating at 60°C under reduced pressure of 1 Torr for 1 hour, producing 1.21 g of the desired Ru(O₂C^{t}Bu)₂((R_{C},R_{C},S_{P},S_{P})-Ph-trap) as an air-stable orange powder. Purity: 96.8% by weight (main impurity was 3.4% by weight of toluene); Isolated Yield: 95.2%. The results of NMR analysis of this complex were consistent with those of its enantiomer, namely Ru(O₂C^{t}Bu)₂((S_{C},S_{C},R_{P},R_{P})-Ph-trap), produced in Example 2. Single crystals of this complex were also successfully prepared in the same manner as its enantiomer.

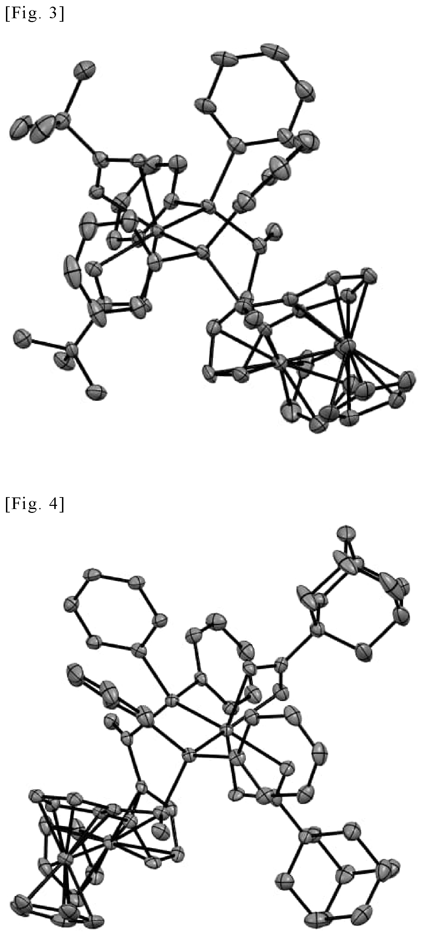

Fig. 3 shows the results of single-crystal X-ray structural analysis (thermal ellipsoid plot; 50% probability level) of Ru(O₂C^{t}Bu)₂((R_{C},R_{C},S_{P},S_{P})-Ph-trap) produced in Example 3. Note that the depiction of hydrogen atoms is omitted from the figure, and the structure of one molecule of this complex is shown in an excerpt for clarity of the relationship with its enantiomer. The result clearly confirmed that this complex is indeed an enantiomer of Ru(O₂C^{t}Bu)₂((S_{C},S_{C},R_{P},R_{P})-Ph-trap), produced in Example 2. The key parameters ensuring the reliability of the results of analysis are summarized below; Chemical Formula: C₁₂₃H₁₃₂Fe₄O₈P₄Ru₂; Crystal System: Monoclinic; Space Group: P1 2₁ 1 (#4); Lattice Constants: a = 11.32740(12) Å, b = 43.9161(4) Å, c = 11.49891(12) Å, α = γ = 90°, β = 111.1986(12)° ; R₁: 0.0415; wR₂: 0.0958; GOF: 1.047; Flack Parameter: -0.015(3).

### [Example 4] Production of Ru(O₂CAd)₂((S_{C},S_{C},R_{P},R_{P})-Ph-trap)

### (Formula 21)

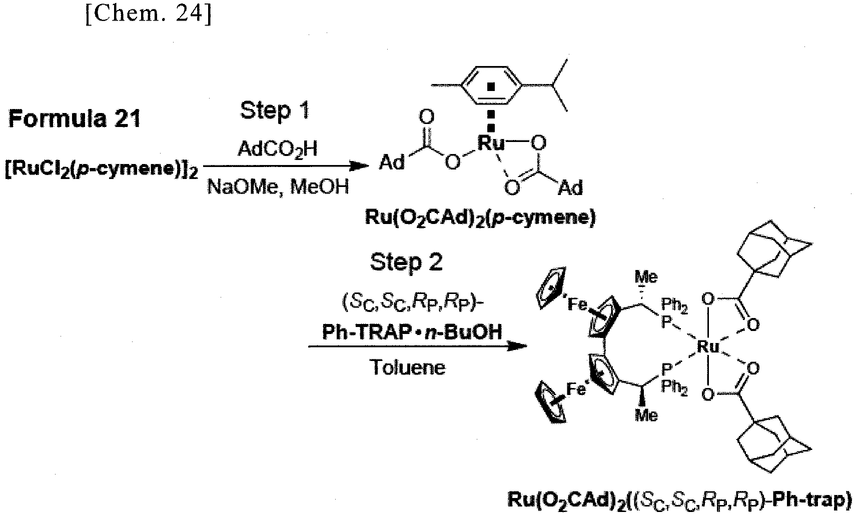

### Step 1: Synthesis of Ru(O₂CAd)₂(p-cymene)

[Setup and Reaction] A 100 mL, four-necked, round-bottomed flask was successively charged with [RuCl₂(p-cymene)]₂ (5.4 g, 8.82 mmol, 1.0 equivalent) and 1-adamantanecarboxylic acid (AdCO₂H; 7.0 g, 38.8 mmol, 4.4 equivalents), equipped with a magnetic stirring bar, thermometer, Dimroth condenser, and a three-way stopcock, and then evacuated and backfilled with nitrogen. Dehydrated MeOH (35 mL) and a MeOH solution of NaOMe (purity: 28.0% by weight, 7.44 mL, 37.0 mmol, 4.2 equivalents) were sequentially added to this flask. The contents of the flask were stirred for 3 hours under reflux while being heated using an oil bath set at 80°C.

[Post-Treatment, Isolation, and Purification] The resulting reddish-orange slurry was cooled to 0°C using an ice-water bath, and then filtered by suction using a Kiriyama funnel to separate crystals from filtrate. The filtered crystals were washed sequentially with MeOH cooled to 0°C (14 mL), tap water (35 mL), and MeOH cooled to 0°C (14 mL), and then dried by heating at 80°C under reduced pressure of 1 Torr for 2 hours, affording 8.16 g of the desired Ru(O₂CAd)₂(p-cymene) as an air-stable orange powder. Purity: 98.5% by weight (main impurity was RuCl(O₂CAd)(p-cymene) as a reaction intermediate); Isolated Yield: 76.7%. Meanwhile, tap water (35 mL) was added to the separated filtrate, and the resulting mixture was stirred for 2 hours at 0°C to give an orange slurry, which was then filtered by suction using a Kiriyama funnel. The filtered crystals were washed with three times with 50% aqueous MeOH (14 mL), and then dried by heating at 80°C under reduced pressure of 1 Torr for 2 hours, affording 2.17 g of the additional Ru(O₂CAd)₂(p-cymene) as a yellowish-orange powder. Purity: 97.1% by weight (main impurity was RuCl(O₂CAd)(p-cymene)); Isolated Yield: 20.1%; Total Yield: 96.8%.

¹H NMR (400 MHz, CDCl₃): δ = 5.72 (d, J = 6.0 Hz, 2H), 5.49 (d, J = 6.0 Hz, 2H), 2.88 (sept, J = 7.2 Hz, 1H), 2.24 (s, 3H), 1.93 (bs, 6H), 1.78 (bs, 12H), 1.70-1.58 (m, 12H), 1.34 (d, J = 7.2 Hz, 6H).
¹³C NMR (100 MHz, CDCl₃): δ = 191.03, 98.57, 93.94, 78.14, 76.80, 41.90, 39.27, 36.71, 31.47, 28.19, 22.48, 18.48.

### Step 2: Production of Ru(O₂CAd)₂((S_{C},S_{C},R_{P},R_{P})-Ph-trap)

[Setup and Reaction] A 100 mL, four-necked, round-bottomed flask was charged with (S_{C},S_{C},R_{P},R_{P})-Ph-TRAP·n-BuOH (purity: 92.6% by weight, 2.5 g, 2.91 mmol, 1.05 equivalents), equipped with a magnetic stirring bar, thermometer, dropping funnel, Claisen distillation apparatus, Dimroth condenser, and a three-way stopcock, and then evacuated and backfilled with nitrogen. Dehydrated toluene (40 mL) was added to this flask, and the resulting orange solution was stirred at 60°C while gradually reducing the pressure inside the apparatus to 60 Torr. After recovering approximately 30 mL of solvent, the inside of the apparatus was filled with nitrogen gas. Ru(O₂CAd)₂(p-cymene) synthesized in Step 1 (purity: 98.5% by weight, 1.67 g, 2.77 mmol, 1.0 equivalent) and dehydrated toluene (30 mL) were sequentially charged into the dropping funnel. The resulting suspension was added dropwise to the reaction solution at room temperature, followed by stirring for 2 hours while being heated using an oil bath set at 70°C.

[Post-Treatment, Isolation, and Purification] The dark-brown reaction solution was stirred at 70°C while gradually reducing the pressure inside the apparatus to 60 Torr. After recovering approximately 33 mL of solvent, the inside of the apparatus was filled with nitrogen gas. The concentrated solution was allowed to cool to room temperature. Dehydrated acetone (30 mL) was added to the resulting brown slurry while being stirred at room temperature. The resulting mixture was cooled to 0°C using an ice-water bath, and then filtered by suction using a Kiriyama funnel under a nitrogen stream. The filtered crystals were washed three times with acetone cooled to 0°C (10 mL), and then dried by heating at 60°C under reduced pressure of 1 Torr for 1 hour, producing 3.14 g of the desired Ru(O₂CAd)₂((S_{C},S_{C},R_{P},R_{P})-Ph-trap) as an air-stable orange powder. Purity: 98.1% by weight (main impurities were 1.4% by weight of toluene and 0.5% by weight of acetone); Isolated Yield: 88.7%. Single crystals of this complex were successfully prepared by solution diffusion using toluene as a good solvent and n-heptane as a poor solvent.

¹H NMR (400 MHz, C₆D₆): δ = 7.68 (bs, 4H), 7.45 (bs, 4H), 7.20 (t, J = 7.2 Hz, 4H), 7.11 (t, J = 7.2 Hz, 2H), 7.08-6.97 (m, 6H [Target] + 0.95H [Toluene]), 4.52 (bs, 2H), 4.11 (t, J = 2.4 Hz, 2H), 4.07 (s, 10H), 3.94-3.60 (bm, 4H), 2.10 (s, 0.57H [Toluene]), 1.93 (bs, 12H), 1.86 (bs, 6H), 1.63-1.51 (bm, 12H [Target] + 0.66H [Acetone]), 1.43 (bs, 6H).
³¹P NMR (161 MHz, C₆D₆): δ = 72.06 (bs, 2P).

Fig. 4 shows the results of single-crystal X-ray structural analysis (thermal ellipsoid plot; 50% probability level) of Ru(O₂CAd)₂((S_{C},S_{C},R_{P},R_{P})-Ph-trap) produced in Example 4. Note that the depiction of hydrogen atoms in the figure is omitted for clarity of the structure of this complex. The results clearly confirmed that: 1) this complex crystallizes without solvation; 2) this complex exhibits C₁ symmetry in its crystalline state; 3) its secondarily induced ruthenium-centered chiral structure is Δ; 4) surprisingly, Ph-TRAP in this complex functions as a cis-chelating chiral diphosphine ligand (bite angle: 104.56°), rather than the conventionally known trans-chelating ligand (bite angle: approximately 180°). The key parameters ensuring the reliability of the results of analysis are summarized below; Chemical Formula: C₇₀H₇₄Fe₂O₄P₂Ru₂; Crystal System: Triclinic; Space Group: P1 (#1); Lattice Constants: a = 12.0194(2) Å, b = 12.0812(2) Å, c = 12.5546(2) Å, α = 62.7282(17)°, β = 65.4211(16)°, γ = 67.5524(16)°; R₁: 0.0438; wR₂: 0.1111; GOF: 1.044; Flack Parameter: -0.024(4).

### [Example 5] Production of Ru(O₂CAd)₂((R_{C},R_{C},S_{P},S_{P})-Ph-trap)

### (Formula 22)

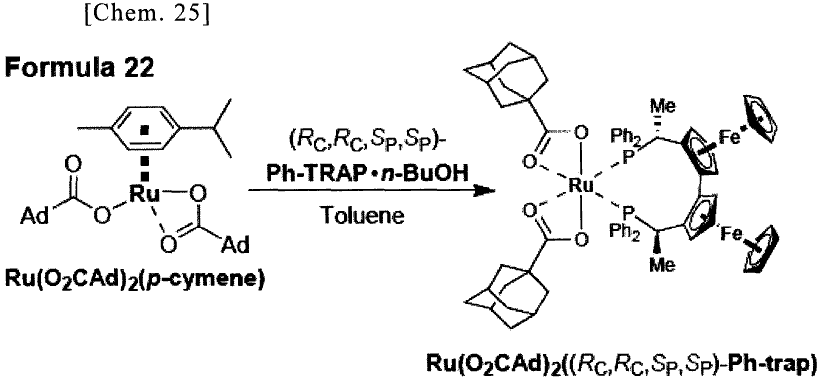

Using (R_{C},R_{C},S_{P},S_{P})-Ph-TRAP·n-BuOH (purity: 93.8% by weight, 1.08 g, 1.28 mmol, 1.05 equivalents) and Ru(O₂CAd)₂(p-cymene) synthesized in Step 1 of Example 4 (purity: 98.5% by weight, 735 mg, 1,22 mmol, 1.0 equivalent) as starting materials, 1.35 g of the desired Ru(O₂CAd)₂((R_{C},R_{C},S_{P},S_{P})-Ph-trap) was produced as an air-stable orange powder by following the procedure described in Example 4. Purity: 98.0% by weight (main impurities were 1.4% by weight of toluene and 0.6% by weight of acetone); Isolated Yield: 86.5%. The results of NMR analysis of this complex were consistent with those of its enantiomer, namely Ru(O₂CAd₂((S_{C},S_{C},R_{P},R_{P})-Ph-trap) produced in Example 5, except for differences in the acetone content. Single crystals of this complex were also successfully prepared in the same manner as its enantiomer.

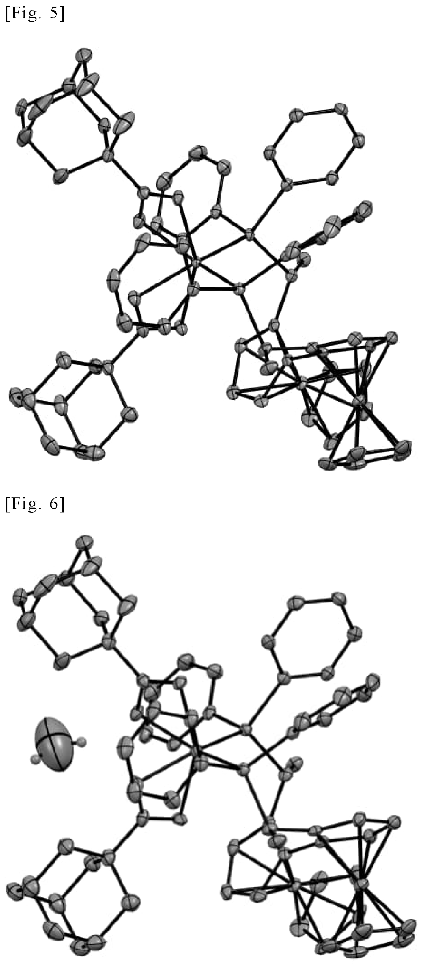

Fig. 5 shows the results of single-crystal X-ray structural analysis (thermal ellipsoid plot; 50% probability level) of Ru(O₂CAd)₂((R_{C},R_{C},S_{P},S_{P})-Ph-trap) produced in Example 5. Note that the depiction of hydrogen atoms is omitted from the figure for clarity of the relationship with its enantiomer. The result clearly confirmed that this complex is indeed an enantiomer of Ru(O₂CAd)₂((S_{C},S_{C},R_{P},R_{P})-Ph-trap), produced in Example 4. The key parameters ensuring the reliability of the results of analysis are summarized below; Chemical Formula: C₇₀H₇₄Fe₂O₄P₂Ru₂; Crystal System: Triclinic; Space Group: P1 (#1); Lattice Constants: a = 12.0321(2) Å, b = 12.07810(10) Å, c = 12.54670(10) Å, α = 62.7470(10)°, β = 65.4940(10)°, γ = 67.5930(10)°; R₁: 0.0518; wR₂: 0.1319; GOF: 1.096; Flack Parameter: -0.012(5).

Meanwhile, single crystals of this complex were also successfully prepared by solution diffusion using toluene as a good solvent and acetone as a poor solvent. Single-crystal X-ray structural analysis revealed that when these single crystals were exposed to air under humid conditions, one equivalent of water was incorporated into each molecule of this complex. Fig. 6 shows the results of single-crystal X-ray structural analysis (thermal ellipsoid plot; 50% probability level) of Ru(O₂CAd)₂((R_{C},R_{C},S_{P},S_{P})-Ph-trap)·H₂O thus obtained. Note that the depiction of hydrogen atoms on the complex molecule is omitted from the figure for clarity of the incorporated water molecule. The key parameters ensuring the reliability of the results of analysis are summarized below; Chemical Formula: C₇₀H₇₆Fe₂O₅P₂Ru₂; Crystal System: Triclinic; Space Group: P1 (#1); Lattice Constants: a = 12.0418(2) Å, b = 12.07310(10) Å, c = 12.5359(2) Å, α = 62.6800(10)°, β = 65.6630(10)°, γ = 67.6240(10)°; R₁: 0.0403; wR₂: 0.1044; GOF: 1.064; Flack Parameter: -0.018(3).

### [Example 6] Production of Ru(O₂CMe)₂((S_{C},S_{C},R_{P},R_{P})-Tol-trap)

### (Formula 23)

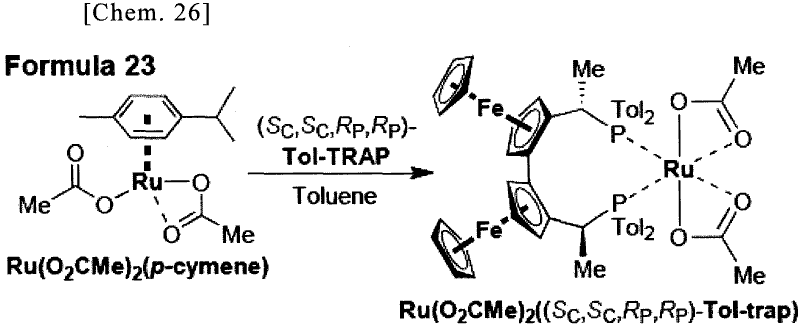

[Setup and Reaction] A 20 mL Schlenk flask was successively charged with (S_{C},S_{C},R_{P},R_{P})-Tol-TRAP (purity: 98.5% by weight, 500 mg, 0.579 mmol, 1.01 equivalents) synthesized according to the method described in International Publication No. WO2024/203802, and Ru(O₂CMe)₂(p-cymene) (203 mg, 0.573 mmol, 1.0 equivalent) synthesized according to Step 1 of Example 1, equipped with a magnetic stirring bar, and then evacuated and backfilled with nitrogen. After adding dehydrated toluene (2.5 mL) to this flask, the contents of the flask were stirred for 2 hours while being heated using an oil bath set at 70°C.

[Post-Treatment and Isolation] The resulting reaction solution was concentrated to dryness by gradually reducing the pressure to 15 Torr while being stirred at 70°C, and then the inside of the flask was filled with nitrogen gas. The resulting dark-brown residue was triturated under a nitrogen atmosphere, and then dried by heating at 70°C under reduced pressure of 1 Torr for 1 hour, producing 680 mg of the desired Ru(O₂CMe)₂((S_{C},S_{C},R_{P},R_{P})-Tol-trap) as a dark-brown powder. Purity: 90.7% by weight (main impurities were 4.5% by weight of toluene and 4.8% by weight of p-cymene); Isolated Yield: Quantitative.

¹H NMR (400 MHz, C₆D₆): δ = 8.06 (bs, 4H), 7.50-7.38 (m, 4H), 7.14-6.97 (m, 2.90H [Toluene] + 1.68H [p-Cymene]), 7.05 (d, J = 8.0 Hz, 4H), 6.89 (d, J = 8.0 Hz, 4H), 4.75 (bs, 2H), 4.32 (bs, 2H), 4.22 (t, J = 2.4 Hz, 2H), 4.15 (bs, 2H), 4.06 (s, 10H), 2.72 (sept, J = 6.8 Hz, 0.42H [p-Cymene]), 2.15 (s, 1.26H [p-Cymene]), 2.10 (s, 1.74H [Toluene]), 2.04 (s, 6H), 1.94 (s, 6H), 1.76 (s, 6H), 1.21 (bs, 6H), 1.15 (d, J = 6.8 Hz, 2.52H [p-Cymene]).
³¹P NMR (161 MHz, C₆D₆): δ = 73.36 (s, 2P).

### [Example 7] Production of Ru(O₂C^{t}Bu)₂((S_{C},S_{C},R_{P},R_{P})-Tol-trap)

### (Formula 24)

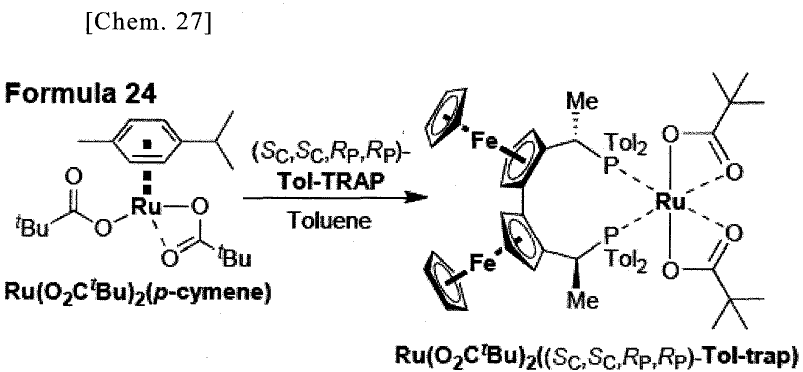

Ru(O₂C^{t}Bu)₂(p-cymene) synthesized in Step 1 of Example 2 (251 mg, 0.573 mmol, 1.0 equivalent) was reacted with (S_{C},S_{C},R_{P},R_{P})-Tol-TRAP (purity: 98.5% by weight, 500 mg, 0.579 mmol, 1.01 equivalents) according to the procedure described in Example 6. Further post-treatment produced 739 mg of the desired Ru(O₂C^{t}Bu)₂((S_{C},S_{C},R_{P},R_{P})-Tol-trap) as a dark-brown powder. Purity: 91.7% by weight (main impurities were 3.0% by weight of toluene and 5.3% by weight of p-cymene); Isolated Yield: Quantitative.

¹H NMR (400 MHz, C₆D₆): δ = 7.62 (bs, 4H), 7.35 (bs, 4H), 7.14-7.00 (m, 2.03H [Toluene] + 2.0H [p-Cymene]), 6.98 (d, J = 8.0 Hz, 4H), 6.84 (d, J = 8.0 Hz, 4H), 4.56 (bs, 2H), 4.14 (t, J = 2.4 Hz, 2H), 4.08 (s, 10H), 4.00 (bs, 2H), 3.82 (bs, 2H), 2.72 (sept, J = 6.8 Hz, 0.5H [p-Cymene]), 2.15 (s, 1.5H [p-Cymene]), 2.10 (s, 1.22H [Toluene]), 2.07 (s, 6H), 2.02 (s, 6H), 1.42 (bs, 6H), 1.23 (s, 18H), 1.15 (d, J = 6.8 Hz, 3.0H [p-Cymene]). ³¹P NMR (161 MHz, C₆D₆): δ = 72.90 (s, 2P).

### [Example 8] Production of Ru(O₂CAd)₂((S_{C},S_{C},R_{P},R_{P})-Tol-trap)

### (Formula 25)

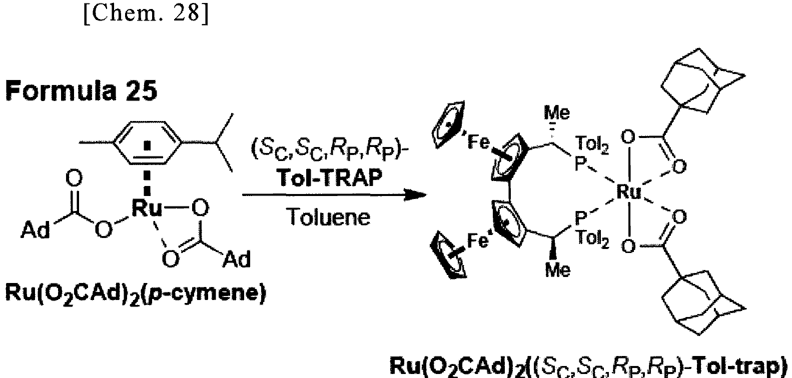

Ru(O₂CAd)₂(p-cymene) synthesized in Step 1 of Example 4 (purity: 98.5% by weight, 346 mg, 0.573 mmol, 1.0 equivalent) was reacted with (S_{C},S_{C},R_{P},R_{P})-Tol-TRAP (purity: 98.5% by weight, 500 mg, 0.579 mmol, 1.01 equivalents) according to the procedure described in Example 6. Further post-treatment produced 842 mg of the desired Ru(O₂C^{t}Bu)₂((S_{C},S_{C},R_{P},R_{P})-Tol-trap) as a dark-brown powder. Purity: 91.4% by weight (main impurities were 3.9% by weight of toluene and 4.7% by weight of p-cymene); Isolated Yield: Quantitative.

¹H NMR (400 MHz, C₆D₆): δ = 7.68 (bs, 4H), 7.39 (bs, 4H), 7.14-6.97 (m, 3.07H [Toluene] + 2.0H [p-Cymene]), 7.05 (d, J = 7.6 Hz, 4H), 6.88 (d, J = 7.6 Hz, 4H), 4.63 (bs, 2H), 4.21 (t, J = 2.4 Hz, 2H), 4.10 (s, 10H), 4.04 (bs, 2H), 3.88 (bs, 2H), 2.72 (sept, J = 6.8 Hz, 0.5H [p-Cymene]), 2.15 (s, 1.5H [p-Cymene]), 2.10 (s, 1.84H [Toluene]), 2.08 (s, 6H), 2.06 (bs, 12H), 2.04 (s, 6H), 1.90 (bs, 6H), 1.68-1.51 (bm, 12H), 1.44 (bs, 6H), 1.15 (d, J = 6.8 Hz, 3.0H [p-Cymene]).
³¹P NMR (161 MHz, C₆D₆): δ = 72.86 (bs, 2P).

### [Example 9] Asymmetric hydrogenation of N-Boc-2-phenyl-1H-indole by using Ru(O₂CMe)₂((S_{C},S_{C},R_{P},R_{P})-Ph-trap) as a catalyst (Formula 26)

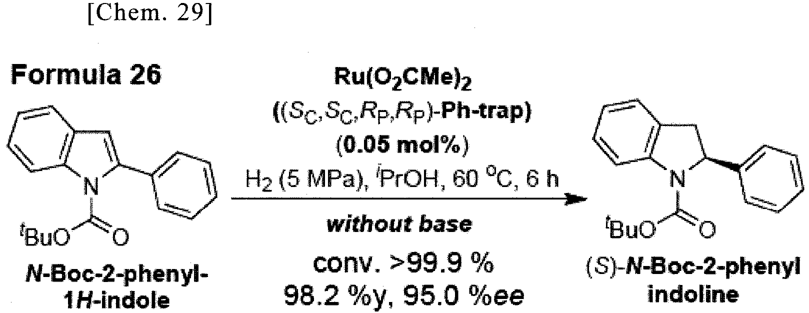

[Setup and Reaction] A borosilicate glass test tube (φ20 mm × 130 mm) was successively charged with Ru(O₂CMe)₂((S_{C},S_{C},R_{P},R_{P})-Ph-trap) produced in Example 1 (purity: 92.1% by weight, 2.8 mg, 0.05 mol%) and N-Boc-2-phenyl-1H-indole synthesized according to the method described in Non-Patent Literature 8 (1.47 g, 5.00 mmol, 1.0 equivalent), and then equipped with a magnetic stirring bar. This test tube was placed in a 50 mL autoclave apparatus, evacuated and backfilled with nitrogen, and subsequently dehydrated isopropyl alcohol ('PrOH; 12 mL) was added. The inside of the apparatus was pressurized to 5 MPa with hydrogen gas, and the contents of the test tube were stirred at 60°C for 6 hours.

[Post-Treatment, Isolation, and Purification] After opening the autoclave apparatus, the reaction solution in the test tube was transferred to a 50 mL round-bottomed flask, and concentrated to dryness under reduced pressure using a rotary evaporator. Analysis of a portion of the resulting residue by ¹H NMR revealed a conversion rate of >99.9%. The residue was purified by silica gel column chromatography (eluent: n-hexane/toluene = 88/12 to 0/100), yielding 1.450 g of the desired (S)-N-Boc-2-phenylindoline as a colorless solid. Isolated Yield: 98.2%, Optical Purity: 95.0%ee. The results of NMR analysis of this compound were consistent with those described in Non-Patent Literature 8. Single crystals of this compound were successfully prepared by solution diffusion using toluene as a good solvent and n-hexane as a poor solvent. The measurement conditions for the optical purity of this compound were as follows; Column: CHIRALPAK IC-3 (manufactured by Daicel Corporation, 4.6 mmφ × 250 mm), Eluent: n-hexane/'PrOH = 99/1, Flow Rate: 0.5 mL/min, Detector: UV 254 nm, Temperature: 20°C, Retention Time: 14.9 min (R-isomer), 21.1 min (S-isomer).

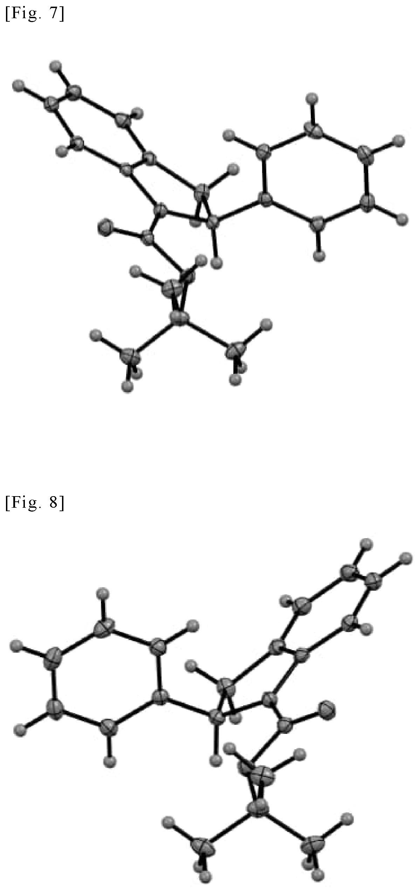

Fig. 7 shows the results of single-crystal X-ray structural analysis (thermal ellipsoid plot; 50% probability level) of (S)-N-Boc-2-phenylindoline synthesized in Example 9. These results determined the previously unknown absolute configuration of this compound (Non-Patent Literature 8) and clarified the steric relationship between the chiral catalyst and its corresponding product. The key parameters ensuring the reliability of the analysis results are summarized below; Chemical Formula: C₁₉H₂₁NO₂, Crystal System: Orthorhombic, Space Group: P2₁2₁2₁ (#19), Lattice Constants: a = 5.85060(4) Å, b = 16.35509(11) Å, c = 16.49422(10) Å, α = β = γ = 90°, R₁: 0.0281, wR₂: 0.0703, GOF: 1.075, Flack parameter: 0.00(6).

### [Example 10] Asymmetric hydrogenation of N-Boc-2-phenyl-1H-indole by using Ru(O₂C^{t}Bu)₂((S_{C},S_{C},R_{P},R_{P})-Ph-trap) as a catalyst

The asymmetric hydrogenation of N-Boc-2-phenyl-1H-indole (1.47 g) was carried out at an internal pressure of 5 MPa according to the procedure described in Example 9 by using Ru(O₂C^{t}Bu)₂((S_{C},S_{C},R_{P},R_{P})-Ph-trap) (purity: 96.8% by weight, 2.8 mg, 0.05 mol%) produced in Example 2 as a catalyst, yielding 1.450 g of the desired (S)-N-Boc-2-phenylindoline. Reaction Time: 2 hours, Conversion Rate: >99.9% (by ¹H NMR analysis), Isolated Yield: 98.2%, Optical Purity: 94.9%ee.

### [Example 11] Asymmetric hydrogenation of N-Boc-2-phenyl-1H-indole by using Ru(O₂CAd)₂((S_{C},S_{C},R_{P},R_{P})-Ph-trap) as a catalyst

The asymmetric hydrogenation of N-Boc-2-phenyl-1H-indole (1.47 g) was carried out at an internal pressure of 5 MPa according to the procedure described in Example 9 by using Ru(O₂CAd)₂((S_{C},S_{C},R_{P},R_{P})-Ph-trap) (purity: 98.1% by weight, 3.2 mg, 0.05 mol%) produced in Example 4 as a catalyst, yielding 1.470 g of the desired (S)-N-Boc-2-phenylindoline. Reaction Time: 1 hour, Conversion Rate : >99.9% (by ¹H NMR analysis), Isolated Yield: 99.5%, Optical Purity: 94.4%ee.

### [Example 12] Low-pressure asymmetric hydrogenation of N-Boc-2-phenyl-1H-indole by using Ru(O₂C^{t}Bu)₂((S_{C},S_{C},R_{P},R_{P})-Ph-trap) as a catalyst

The asymmetric hydrogenation of N-Boc-2-phenyl-1H-indole (1.47 g) was carried out at an internal pressure of 1 MPa according to the procedure described in Example 9 by using Ru(O₂C^{t}Bu)₂((S_{C},S_{C},R_{P},R_{P})-Ph-trap) (0.05 mol%) produced in Example 2 as a catalyst, yielding 1.453 g of the desired (S)-N-Boc-2-phenylindoline. Reaction Time: 6 hours, Conversion Rate: >99.9% (by ¹H NMR analysis), Isolated Yield: 98.4%, Optical Purity: 94.7%ee.

### [Example 13] Low-pressure asymmetric hydrogenation of N-Boc-2-phenyl-1H-indole by using Ru(O₂CAd)₂((S_{C},S_{C},R_{P},R_{P})-Ph-trap) as a catalyst

The asymmetric hydrogenation of N-Boc-2-phenyl-1H-indole (1.47 g) was carried out at an internal pressure of 1 MPa according to the procedure described in Example 9 by using Ru(O₂CAd)₂((S_{C},S_{C},R_{P},R_{P})-Ph-trap) (0.05 mol%) produced in Example 4 as a catalyst, yielding 1.456 g of the desired (S)-N-Boc-2-phenylindoline. Reaction Time: 6 hours, Conversion Rate: >99.9% (by ¹H NMR analysis), Isolated Yield: 98.6%, Optical Purity: 94.9%ee.

### [Comparative Example 1] Attempt at asymmetric hydrogenation of N-Boc-2-phenyl-1H-indole by using [RuCl(p-cymene)((S_{C},S_{C},R_{P},R_{P})-Ph-trap)]Cl as a catalyst

The asymmetric hydrogenation of N-Boc-2-phenyl-1H-indole was carried out according to the procedure described in Example 9 by using the conventional catalyst, namely [RuCl(p-cymene)((S_{C},S_{C},R_{P},R_{P})-Ph-trap)]Cl (0.05 mol%), synthesized according to the method described in Non-Patent Literature 8; however, even after carrying out the reaction for 6 hours at an internal pressure of 5 MPa, the conversion rate was only 2.2% (by ¹H NMR analysis).

### [Comparative Example 2] Attempt at asymmetric hydrogenation of N-Boc-2-phenyl-1H-indole by using Ru(O₂CMe)₂((S)-binap) as a catalyst

The asymmetric hydrogenation of N-Boc-2-phenyl-1H-indole was attempted according to the procedure described in Example 9 by using the commercially available common catalyst, namely Ru(O₂CMe)₂((S)-binap) (0.05 mol%); however, even after carrying out the reaction for 6 hours at an internal pressure of 5 MPa, the conversion rate was less than 0.1% (by ¹H NMR analysis).

Table 1 below summarizes the results of Comparative Examples 1 to 2 and Examples 9 to 13. For reference, Formula 27 below shows the stereochemical structures of the various catalysts used in these Comparative Examples and Examples.

**[Table 1]**

| Example/ Comparative Example | Hydrogen Pressure (MPa) | Reaction Time (h) | Conversion Rate (%) | Isolated Yield (%) | Optical Purity (%ee) |
|---|---|---|---|---|---|
| Comparative Example 1 | 5 | 6 | 2.2 | - | - |
| Comparative Example 2 | 5 | 6 | <0.1 | - | - |
| Example 9 | 5 | 6 | >99.9 | 98.2 | 95.0 |
| Example 10 | 5 | 2 | >99.9 | 98.2 | 94.9 |
| Example 11 | 5 | 1 | >99.9 | 99.5 | 94.4 |
| Example 12 | 1 | 6 | >99.9 | 98.4 | 94.7 |
| Example 13 | 1 | 6 | >99.9 | 98.6 | 94.9 |

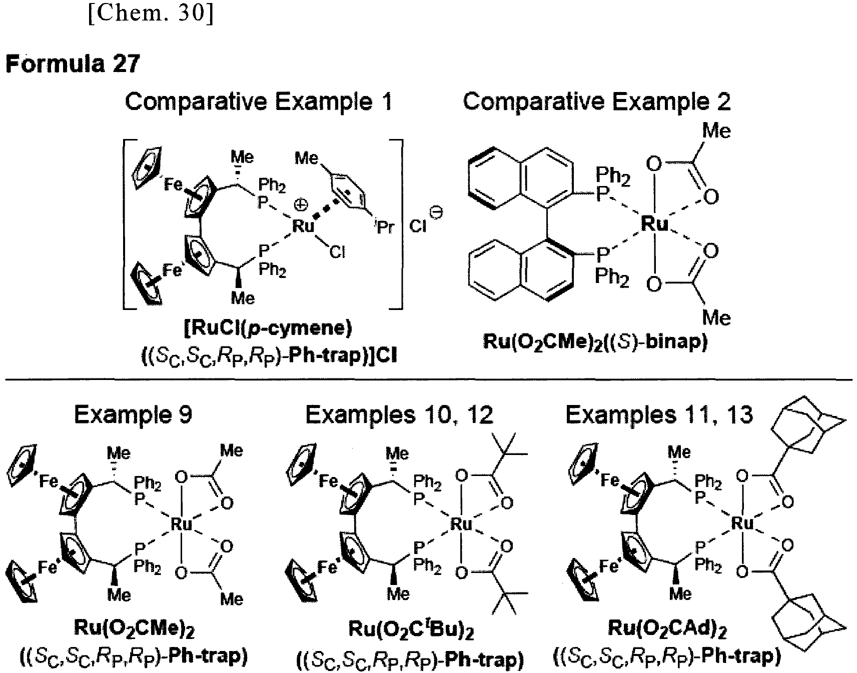

As is clear from the results summarized in Table 1, when using the conventional [RuCl(p-cymene)((S_{C},S_{C},R_{P},R_{P})-Ph-trap)]Cl or the common Ru(O₂CMe)₂((S)-binap) as a catalyst, the asymmetric hydrogenation of N-Boc-2-phenyl-1H-indole, a type of bulky, unreactive heteroaromatic compound, hardly proceeded without the addition of a base (Comparative Example 1 and Comparative Example 2).

On the other hand, when Ru(O₂CMe)₂((S_{C},S_{C},R_{P},R_{P})-Ph-trap), a preferred form of the ruthenium complex (1) of the present invention, was used as a catalyst, the desired reaction proceeded smoothly without the addition of a base, and (S)-N-Boc-2-phenylindoline, a type of optically active cyclic compound, was quantitatively obtained with excellent optical purity (Example 9). These results revealed that the coexistence of the acetate ligand (O₂CMe) and (S_{C},S_{C},R_{P},R_{P})-Ph-TRAP on a divalent ruthenium ion (Ru) enables the catalytic activity of this complex to be exhibited even under neutral conditions.

In addition, by replacing the acetate ligand (O₂CMe) on this complex with a bulkier pivalate ligand (O₂C^{t}Bu) or a 1-adamantanecarboxylate ligand (O₂CAd), the reaction time was significantly reduced while maintaining excellent optical purity and quantitative yield of the product (Example 10 and Example 11). Furthermore, by using these highly active catalysts, the practical low-pressure asymmetric hydrogenation of aromatics was successfully completed within 6 hours even when the internal pressure was reduced from 5 MPa to 1 MPa (Example 12 and Example 13). These results clearly demonstrate that the ruthenium complex (1) of the present invention is superior to the conventional and common catalysts.

### [Example 14] Low-pressure asymmetric hydrogenation of N-Boc-2-phenyl-1H-indole by using Ru(O₂CAd)₂((R_{C},R_{C},S_{P},S_{P})-Ph-trap) as a catalyst (Formula 28)

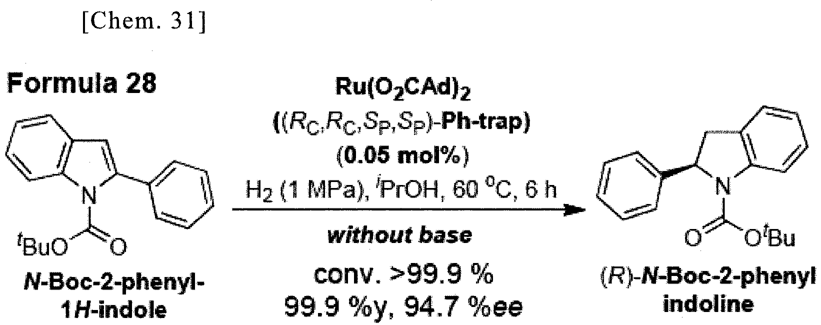

The asymmetric hydrogenation of N-Boc-2-phenyl-1H-indole (1.47 g) was carried out at an internal pressure of 1 MPa according to the procedure described in Example 9 by using Ru(O₂CAd)₂((R_{C},R_{C},S_{P},S_{P})-Ph-trap) (purity: 98.0% by weight, 3.2 mg, 0.05 mol%) produced in Example 5 as a catalyst, yielding 1.475 g of the desired (R)-N-Boc-2-phenylindoline. Reaction Time: 6 hours, Conversion Rate: >99.9% (by ¹H NMR analysis), Isolated Yield: 99.9%, Optical Purity: 94.7%ee. The results of NMR analysis of this compound were consistent with those of its enantiomer, namely (R)-N-Boc-2-phenylindoline, synthesized in Example 9. Single crystals of this compound were also successfully prepared in the same manner as its enantiomer.

Fig. 8 shows the results of single-crystal X-ray structural analysis (thermal ellipsoid plot; 50% probability level) of (R)-N-Boc-2-phenylindoline synthesized in Example 14. These results clearly confirmed that this compound is indeed an enantiomer of (S)-N-Boc-2-phenylindoline, synthesized in Example 9. The key parameters ensuring the reliability of the analysis results are summarized below; Chemical Formula: C₁₉H₂₁NO₂, Crystal System: Orthorhombic, Space Group: P2₁2₁2₁ (#19), Lattice Constants: a = 5.85762(4) Å, b = 16.36040(11) Å, c = 16.51152(10) Å, α = β = γ = 90°, R₁: 0.0290, wR₂: 0.0733, GOF: 1.080, Flack parameter: -0.07(4).

As is clear from Example 13 and Example 14, it is possible to produce both enantiomers of the desired optically active cyclic compound by performing the asymmetric hydrogenation of aromatics by using both enantiomers of the ruthenium complex (1) of the present invention. These results clearly demonstrate that the ruthenium complex (1) of the present invention is practically applicable in the asymmetric hydrogenation of aromatics.

### [Example 15] Asymmetric hydrogenation of methyl N-Boc-1H-indole-2-carboxylate by using Ru(O₂CMe)₂((S_{C},S_{C},R_{P},R_{P})-Ph-trap) as a catalyst (Formula 29)

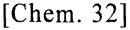

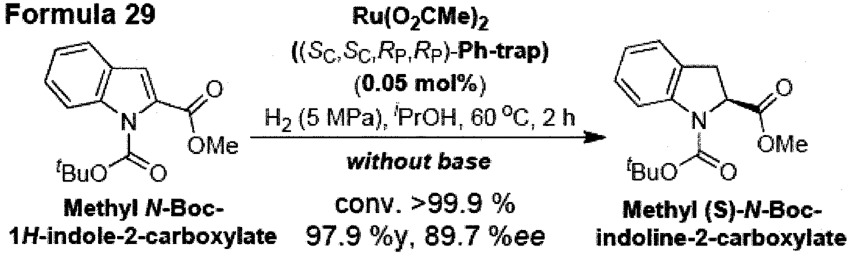

[Setup and Reaction] A borosilicate glass test tube (φ20 mm × 130 mm) was successively charged with Ru(O₂CMe)₂((S_{C},S_{C},R_{P},R_{P})-Ph-trap) produced in Example 1 (purity: 92.1% by weight, 2.8 mg, 0.05 mol%) and methyl N-Boc-1H-indole-2-carboxylate synthesized according to the method described in Non-Patent Literature 8 (1.38 g, 5.00 mmol, 1.0 equivalent), and then equipped with a magnetic stirring bar. This test tube was placed in a 50 mL autoclave apparatus, evacuated and backfilled with nitrogen, and subsequently dehydrated ⁱPrOH (14 mL) was added. The inside of the apparatus was pressurized to 5 MPa with hydrogen gas, and the contents of the test tube were stirred at 60°C for 2 hours.

[Post-Treatment, Isolation, and Purification] After opening the autoclave apparatus, the reaction solution in the test tube was transferred to a 50 mL round-bottomed flask, and concentrated to dryness under reduced pressure using a rotary evaporator. Analysis of a portion of the resulting residue by ¹H NMR revealed a conversion rate of >99.9%. The residue was purified by silica gel column chromatography (eluent: n-hexane/ethyl acetate = 98/2 to 82/18), yielding 1.357 g of the desired methyl (S)-N-Boc-indoline-2-carboxylate as a colorless solid. Isolated Yield: 97.9%, Optical Purity: 89.7%ee. The results of NMR analysis of this compound were consistent with those described in Non-Patent Literature 8. The measurement conditions for the optical purity of this compound were as follows; Column: CHIRALPAK AD (manufactured by Daicel Corporation, 4.6 mmφ × 250 mm), Eluent: n-hexane/ⁱPrOH = 90/10, Flow Rate: 0.5 mL/min, Detector: UV 254 nm, Temperature: 20°C, Retention Time: 9.9 min (R-isomer), 12.0 min (S-isomer).

### [Comparative Example 3] Attempt at asymmetric hydrogenation of methyl N-Boc-1H-indole-2-carboxylate by using a conventional catalyst

The asymmetric hydrogenation of methyl N-Boc-1H-indole-2-carboxylate was carried out according to the procedure described in Example 15 by using the conventional catalyst, namely [RuCl(p-cymene)((S_{C},S_{C},R_{P},R_{P})-Ph-trap)]Cl (0.05 mol%); however, the conversion rate was only 4.1% (by ¹H NMR analysis).

As is clear from Example 15 and Comparative Example 3, by using a preferred form of the ruthenium complex (1) of the present invention instead of the conventional catalyst, the asymmetric hydrogenation of indoles having functional groups that are prone to decompose under strongly basic conditions proceeded with high stereo selectivity, resulting in the quantitative production of industrially useful optically active cyclic amino acid derivatives. These results clearly demonstrate the usefulness of the ruthenium complex (1) of the present invention, which exhibits excellent catalytic activity without the addition of a base.

### [Example 16] Asymmetric hydrogenation of N-Boc-3-methyl-1H-indole by using Ru(O₂CAd)₂((S_{C},S_{C},R_{P},R_{P})-Ph-trap) as a catalyst (Formula 30)

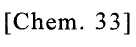

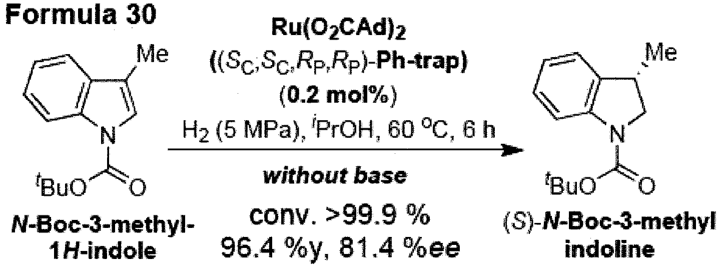

[Setup and Reaction] A borosilicate glass test tube (φ20 mm × 130 mm) was sequentially charged with N-Boc-3-methyl-1H-indole synthesized according to the method described in Non-Patent Literature 8 (purity: 99.6% by weight, 500 mg, 2.15 mmol, 1.0 equivalent) and Ru(O₂CAd)₂((S_{C},S_{C},R_{P},Rₚ)-Ph-trap) produced in Example 4 (purity: 98.1% by weight, 5.5 mg, 0.2 mol%), and then equipped with a magnetic stirring bar. This test tube was placed in a 50 mL autoclave apparatus, evacuated and backfilled with nitrogen, and subsequently dehydrated 'PrOH (5 mL) was added. The inside of the apparatus was pressurized to 5 MPa with hydrogen gas, and the contents of the test tube were stirred at 60°C for 6 hours.

[Post-Treatment, Isolation, and Purification] After opening the autoclave apparatus, the reaction solution in the test tube was transferred to a 50 mL round-bottomed flask, and concentrated to dryness under reduced pressure using a rotary evaporator. Analysis of a portion of the resulting residue by ¹H NMR revealed a conversion rate of >99.9%. The residue was purified by silica gel column chromatography (eluent: n-hexane/ethyl acetate = 98/2 to 85/15), yielding 484 mg of the desired (S)-N-Boc-3-methylindoline as a colorless viscous liquid. Isolated Yield: 96.4%, Optical Purity: 81.4%ee. The results of NMR analysis of this compound were consistent with those described in Non-Patent Literature 8. The measurement conditions for the optical purity of this compound were as follows; Column: CHIRALPAK IC-3 (manufactured by Daicel Corporation, 4.6 mmφ × 250 mm), Eluent: n-hexane/'PrOH = 99/1, Flow Rate: 0.5 mL/min, Detector: UV 254 nm, Temperature: 20°C, Retention Time: 13.8 min (S-isomer), 15.1 min (R-isomer).

### [Comparative Example 4] Attempt at asymmetric hydrogenation of N-Boc-3-methyl-1H-indole by using a conventional catalyst

The asymmetric hydrogenation of N-Boc-3-methyl-1H-indole was attempted according to the procedure described in Example 16 by using the conventional catalyst, namely [RuCl(p-cymene)((S_{C},S_{C},R_{P},R_{P})-Ph-trap)]Cl (0.2 mol%); however, the conversion rate was less than 0.1% (by ¹H NMR analysis).

### [Example 17] Asymmetric hydrogenation of N-Boc-3-phenyl-1H-indole by using Ru(O₂CAd)₂((S_{C},S_{C},R_{P},R_{P})-Ph-trap) as a catalyst (Formula 31)

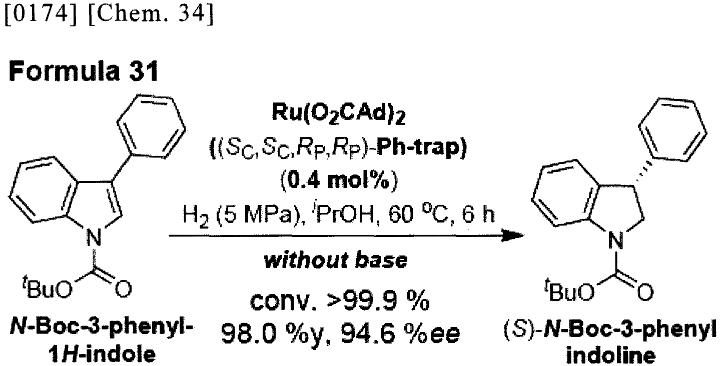

[Setup and Reaction] A borosilicate glass test tube (φ20 mm × 130 mm) was successively charged with Ru(O₂CAd)₂((S_{C},S_{C},R_{P},R_{P})-Ph-trap) produced in Example 4 (purity: 98.1% by weight, 5.5 mg, 0.4 mol%) and N-Boc-3-phenyl-1H-indole synthesized according to the method described in Non-Patent Literature 8 (316 mg, 1.08 mmol, 1.0 equivalent), and then equipped with a magnetic stirring bar. This test tube was placed in a 50 mL autoclave apparatus, evacuated and backfilled with nitrogen, and subsequently dehydrated ⁱPrOH (3 mL) was added. The inside of the apparatus was pressurized to 5 MPa with hydrogen gas, and the contents of the test tube were stirred at 60°C for 6 hours.

[Post-Treatment, Isolation, and Purification] After opening the autoclave apparatus, the reaction solution in the test tube was transferred to a 50 mL round-bottomed flask, and concentrated to dryness under reduced pressure using a rotary evaporator. Analysis of a portion of the resulting residue by ¹H NMR revealed a conversion rate of >99.9%. The residue was purified by silica gel column chromatography (eluent: n-hexane/toluene = 88/12 to 0/100), yielding 313 mg of the desired (S)-N-Boc-3-phenylindoline as a colorless solid. Isolated Yield: 98.0%, Optical Purity: 94.6%ee. The results of NMR analysis of this compound were consistent with those described in Non-Patent Literature 8. Single crystals of this compound were successfully prepared by solution diffusion using toluene as a good solvent and n-hexane as a poor solvent. The measurement conditions for the optical purity of this compound were as follows; Column: CHIRALPAK AD-H (manufactured by Daicel Corporation, 4.6 mmφ × 250 mm), Eluent: n-hexane/'PrOH = 99/1, Flow Rate: 0.5 mL/min, Detector: UV 254 nm, Temperature: 20°C, Retention Time: 11.3 min (S-isomer), 13.2 min (R-isomer).

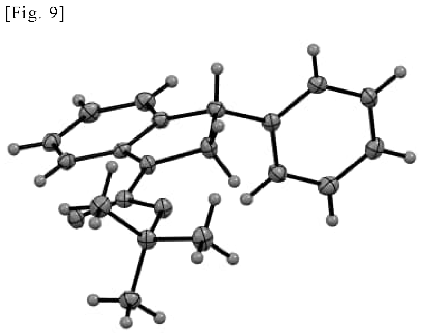

Fig. 9 shows the results of single crystal X-ray structural analysis (thermal ellipsoid plot; 50% probability level) of (S)-N-Boc-3-phenylindoline synthesized in Example 17. These results determined the previously unknown absolute configuration of this compound (Non-Patent Literature 8) and clarified the steric relationship between the chiral catalyst and its corresponding product. The key parameters ensuring the reliability of the analysis results are summarized below; Chemical Formula: C₁₉H₂₁NO₂, Crystal System: Orthorhombic, Space Group: P2₁2₁2₁ (#19), Lattice Constants: a = 8.85782(7) Å, b = 11.09725(9) Å, c = 16.08239(13) Å, α = β = γ = 90°, R₁: 0.0305, wR₂: 0.0765, GOF: 1.021, Flack parameter: -0.05(8).

### [Comparative Example 5] Attempt at asymmetric hydrogenation of N-Boc-3-phenyl-1H-indole by using a conventional catalyst

The asymmetric hydrogenation of N-Boc-3-phenyl-1H-indole was attempted according to the procedure described in Example 17 by using the conventional catalyst, namely [RuCl(p-cymene)((S_{C},S_{C},R_{P},R_{P})-Ph-trap)]Cl (0.4 mol%); however, the conversion rate was less than 0.1% (by ¹H NMR analysis).

### [Example 18] Asymmetric hydrogenation of 2,4-diphenyloxazole by using Ru(O₂CAd)₂((S_{C},S_{C},R_{P},R_{P})-Ph-trap) as a catalyst (Formula 32)

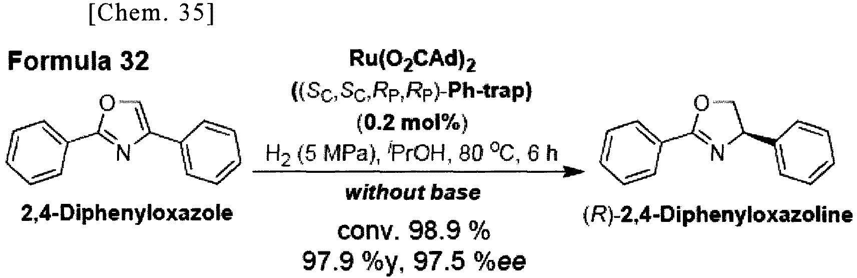

[Setup and Reaction] A borosilicate glass test tube (φ20 mm × 130 mm) was successively charged with Ru(O₂CAd)₂((S_{C},S_{C},R_{P},R_{P})-Ph-trap) produced in Example 4 (purity: 98.1% by weight, 2.9 mg, 0.2 mol%) and commercially available 2,4-diphenyloxazole (250 mg, 1.13 mmol, 1.0 equivalent), and then equipped with a magnetic stirring bar. This test tube was placed in a 50 mL autoclave apparatus, evacuated and backfilled with nitrogen, and subsequently dehydrated ⁱPrOH (2.5 mL) was added. The inside of the apparatus was pressurized to 5 MPa with hydrogen gas, and the contents of the test tube were stirred at 80°C for 6 hours.

[Post-Treatment, Isolation, and Purification] After opening the autoclave apparatus, the reaction solution in the test tube was transferred to a 50 mL round-bottomed flask, and concentrated to dryness under reduced pressure using a rotary evaporator. Analysis of a portion of the resulting residue by ¹H NMR revealed a conversion rate of 98.9%. The residue was purified by silica gel column chromatography (eluent: n-hexane/ethyl acetate = 96/4 to 70/30), yielding 247 mg of the desired (R)-2,4-diphenyloxazoline as a colorless viscous liquid. Isolated Yield: 97.9%, Optical Purity: 97.5%ee. The results of NMR analysis of this compound were consistent with those described in Non-Patent Literature 3. The measurement conditions for the optical purity of this compound were as follows; Column: CHIRALPAK IC-3 (manufactured by Daicel Corporation, 4.6 mmφ × 250 mm), Eluent: n-hexane/'PrOH = 90/10, Flow Rate: 0.5 mL/min, Detector: UV 254 nm, Temperature: 20°C, Retention Time: 13.7 min (R-isomer), 16.7 min (S-isomer).

### [Comparative Example 6] Attempt at asymmetric hydrogenation of 2,4-diphenyloxazole by using a conventional catalyst

The asymmetric hydrogenation of 2,4-diphenyloxazole was attempted according to the procedure described in Example 18 by using the conventional catalyst, namely [RuCl(p-cymene)((S_{C},S_{C},R_{P},R_{P})-Ph-trap)]Cl (0.2 mol%); however, the conversion rate was less than 0.1% (by ¹H NMR analysis).

### [Example 19] Asymmetric hydrogenation of 2,5-diphenyloxazole by using Ru(O₂CAd)₂((S_{C},S_{C},R_{P},R_{P})-Ph-trap) as a catalyst (Formula 33)

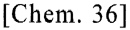

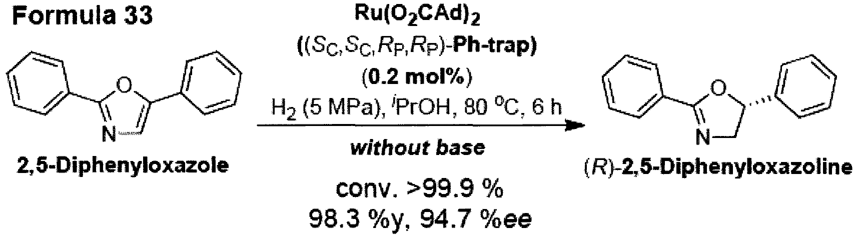

[Setup and Reaction] A borosilicate glass test tube (φ20 mm × 130 mm) was successively charged with Ru(O₂CAd)₂((S_{C},S_{C},R_{P},R_{P})-Ph-trap) produced in Example 4 (purity: 98.1% by weight, 2.9 mg, 0.2 mol%) and commercially available 2,5-diphenyloxazole (250 mg, 1.13 mmol, 1.0 equivalent), and then equipped with a magnetic stirring bar. This test tube was placed in a 50 mL autoclave apparatus, evacuated and backfilled with nitrogen, and subsequently dehydrated ⁱPrOH (2.5 mL) was added. The inside of the apparatus was pressurized to 5 MPa with hydrogen gas, and the contents of the test tube were stirred at 80°C for 6 hours.

[Post-Treatment, Isolation, and Purification] After opening the autoclave apparatus, the reaction solution in the test tube was transferred to a 50 mL round-bottomed flask, and concentrated to dryness under reduced pressure using a rotary evaporator. Analysis of a portion of the resulting residue by ¹H NMR revealed a conversion rate of >99.9%. The residue was purified by silica gel column chromatography (eluent: n-hexane/ethyl acetate = 94/6 to 50/50), yielding 248 mg of the desired (R)-2,5-diphenyloxazoline as a colorless viscous liquid. Isolated Yield: 98.3%, Optical Purity: 94.7%ee. The results of NMR analysis of this compound were consistent with those described in Non-Patent Literature 3. The measurement conditions for the optical purity of this compound were as follows; Column: CHIRALPAK IC-3 (manufactured by Daicel Corporation, 4.6 mmφ × 250 mm), Eluent: n-hexane/'PrOH = 90/10, Flow Rate: 0.5 mL/min, Detector: UV 254 nm, Temperature: 20°C, Retention Time: 15.6 min (S-isomer), 17.0 min (R-isomer).

### [Comparative Example 7] Attempt at asymmetric hydrogenation of 2,5-diphenyloxazole by using a conventional catalyst

The asymmetric hydrogenation of 2,5-diphenyloxazole was attempted according to the procedure described in Example 19 by using the conventional catalyst, namely [RuCl(p-cymene)((S_{C},S_{C},R_{P},R_{P})-Ph-trap)]Cl (0.2 mol%); however, the conversion rate was less than 0.1% (by ¹H NMR analysis).

### [Example 20] Asymmetric hydrogenation of 1-Boc-4-methyl-2-phenylimidazole by using Ru(O₂CAd)₂((S_{C},S_{C},R_{P},R_{P})-Ph-trap) as a catalyst (Formula 34)

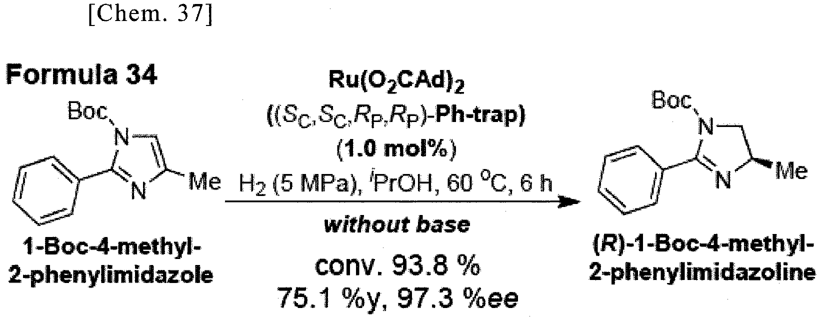

[Setup and Reaction] A borosilicate glass test tube (φ20 mm × 130 mm) was successively charged with 1-Boc-4-methyl-2-phenylimidazole synthesized according to the method described in Non-Patent Literature 3 (303 mg, 1.17 mmol, 1.0 equivalent) and Ru(O₂CAd)₂((S_{C},S_{C},R_{P},R_{P})-Ph-trap) produced in Example 4 (purity: 98.1% by weight, 15.0 mg, 1.0 mol%), and then equipped with a magnetic stirring bar. This test tube was placed in a 50 mL autoclave apparatus, evacuated and backfilled with nitrogen, and subsequently dehydrated ⁱPrOH (3 mL) was added. The inside of the apparatus was pressurized to 5 MPa with hydrogen gas, and the contents of the test tube were stirred at 60°C for 6 hours.

[Post-Treatment, Isolation, and Purification] After opening the autoclave apparatus, the reaction solution in the test tube was transferred to a 50 mL round-bottomed flask, and concentrated to dryness under reduced pressure using a rotary evaporator. Analysis of a portion of the resulting residue by ¹H NMR revealed a conversion rate of 93.8%. The residue was purified by silica gel column chromatography (eluent: n-hexane/ethyl acetate = 95/5 to 50/50), yielding 229 mg of the desired (R)-1-Boc-4-methyl-2-phenylimidazoline as a colorless viscous liquid. Isolated Yield: 75.1%, Optical Purity: 97.3%ee. The results of NMR analysis of this compound were consistent with those described in Non-Patent Literature 3. The measurement conditions for the optical purity of this compound were as follows; Column: CHIRALPAK IC-3 (manufactured by Daicel Corporation, 4.6 mmφ × 250 mm), Eluent: n-hexane/'PrOH = 90/10, Flow Rate: 0.5 mL/min, Detector: UV 254 nm, Temperature: 20°C, Retention Time: 24.8 min (R-isomer), 26.5 min (S-isomer).

### [Comparative Example 8] Attempt at asymmetric hydrogenation of 1-Boc-4-methyl-2-phenylimidazole by using a conventional catalyst

The asymmetric hydrogenation of 1-Boc-4-methyl-2-phenylimidazole was attempted according to the procedure described in Example 20 by using the conventional catalyst, namely [RuCl(p-cymene)((S_{C},S_{C},R_{P},R_{P})-Ph-trap)]Cl (1.0 mol%); however, the substrate decomposed while the Boc group dissociated in this reaction system.

### [Example 21] Asymmetric hydrogenation of diisobutyl naphthalene-2,6-dicarboxylate by using Ru(O₂CAd)₂((S_{C},S_{C},R_{P},R_{P})-Ph-trap) as a catalyst (Formula 35)

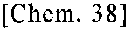

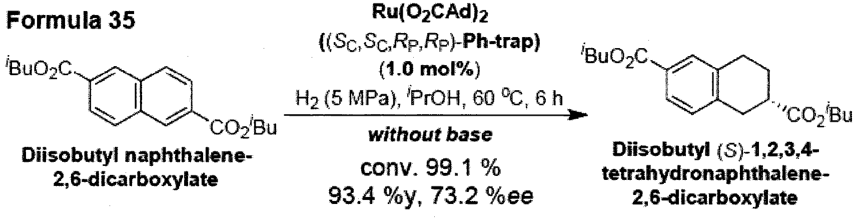

[Setup and Reaction] A borosilicate glass test tube (φ20 mm × 130 mm) was successively charged with Ru(O₂CAd)₂((S_{C},S_{C},R_{P},R_{P})-Ph-trap) produced in Example 4 (purity: 98.1% by weight, 11.7 mg, 1.0 mol%) and diisobutyl naphthalene-2,6-dicarboxylate synthesized according to the method described in Non-Patent Literature 9 (300 mg, 0.913 mmol, 1.0 equivalent), and then equipped with a magnetic stirring bar. This test tube was placed in a 50 mL autoclave apparatus, evacuated and backfilled with nitrogen, and subsequently dehydrated ⁱPrOH (4.5 mL) was added. The inside of the apparatus was pressurized to 5 MPa with hydrogen gas, and the contents of the test tube were stirred at 60°C for 6 hours.

[Post-Treatment, Isolation, and Purification] After opening the autoclave apparatus, the reaction solution in the test tube was transferred to a 50 mL round-bottomed flask, and concentrated to dryness under reduced pressure using a rotary evaporator. Analysis of a portion of the resulting residue by ¹H NMR revealed a conversion rate of 99.1%. The residue was purified by silica gel column chromatography (eluent: n-hexane/ethyl acetate = 98/2 to 82/18), yielding 283 mg of the desired diisobutyl (S)-1,2,3,4-tetrahydronaphthalene-2,6-dicarboxylate as a colorless viscous liquid. Isolated Yield: 93.4%, Optical Purity: 73.2%ee. The results of NMR analysis of this compound were consistent with those described in Non-Patent Literature 9. The measurement conditions for the optical purity of this compound were as follows; Column: CHIRALPAK IC-3 (manufactured by Daicel Corporation, 4.6 mmφ × 250 mm), Eluent: n-hexane/'PrOH = 99/1, Flow Rate: 0.5 mL/min, Detector: UV 254 nm, Temperature: 20°C, Retention Time: 7.3 min (R-isomer), 9.8 min (S-isomer).

### [Comparative Example 9] Attempt at asymmetric hydrogenation of diisobutyl naphthalene-2,6-dicarboxylate by using a conventional catalyst

The asymmetric hydrogenation of diisobutyl naphthalene-2,6-dicarboxylate was attempted according to the procedure described in Example 21 by using the conventional catalyst, namely [RuCl(p-cymene)((S_{C},S_{C},R_{P},R_{P})-Ph-trap)]Cl (1.0 mol%); however, the conversion rate was less than 0.1% (by ¹H NMR analysis).

Table 2 below summarizes the results of Examples 16 to 21 and Comparative Examples 4 to 9 for each substrate. For reference, Formula 36 below shows the stereochemical structures of the catalysts used in these Examples and Comparative Examples.

**[Table 2]**

| Example/ Comparative Example | Catalyst Amount (mol%) | Reaction Temp. (°C) | Conversion Rate (%) | Isolated Yield (%) | Optical Purity (%ee) |
|---|---|---|---|---|---|
| Example 16 | 0.2 | 60 | >99.9 | 96.4 | 81.4 |
| Comparative Example 4 | | | <0.1 | - | - |
| Example 17 | 0.4 | 60 | >99.9 | 98.0 | 94.6 |
| Comparative Example 5 | | | <0.1 | - | - |
| Example 18 | 0.2 | 80 | 98.9 | 97.9 | 97.5 |
| Comparative Example 6 | | | <0.1 | - | - |
| Example 19 | 0.2 | 80 | >99.9 | 98.3 | 94.7 |
| Comparative Example 7 | | | <0.1 | - | - |
| Example 20 | 1.0 | 60 | 93.8 | 75.1 | 97.3 |
| Comparative Example 8 | | | Decomp. | - | - |
| Example 21 | 1.0 | 60 | 99.1 | 93.4 | 73.2 |
| Comparative Example 9 | | | <0.1 | - | - |

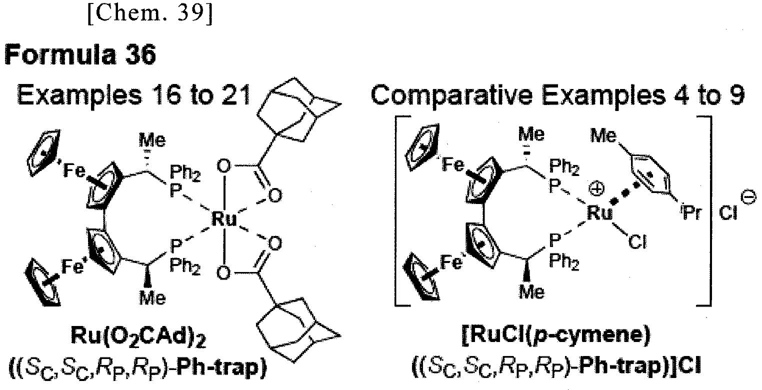

As is clear from the results summarized in Table 2, when Ru(O₂CAd)₂((S_{C},S_{C},R_{P},R_{P})-Ph-trap), a particularly preferred form of the ruthenium complex (1) of the present invention, was used as a catalyst, the asymmetric hydrogenation of various aromatic and heteroaromatic compounds, such as indoles, oxazoles, imidazoles, and naphthalenes, proceeded smoothly without the addition of a base, producing industrially useful optically active cyclic compounds in good yield (Examples 16 to 21). Instead, when using the conventional catalyst, namely [RuCl(p-cymene)((S_{C},S_{C},R_{P},R_{P})-Ph-trap)]Cl, the desired reaction did not proceed without the addition of a base, and some of the substrates were decomposed (Comparative Examples 4 to 9). These results clearly demonstrate the usefulness of the ruthenium complex (1) of the present invention, which does not require a base and has an excellent range of substrate applicability.

### Industrial Applicability

The ruthenium complex (1) of the present invention can be readily produced by reacting R^{P}-TRAP (2) with a ruthenium source (3). In addition, the preferred form of the ruthenium complex (1) of the invention has excellent crystallinity, facilitating isolation, purification, and long-term storage. Furthermore, this preferred form exhibits excellent catalytic activity, asymmetric induction ability, and substrate generality even without the addition of a base. Accordingly, the present invention can contribute to improving the efficiency and facilitating the practical application of various organic synthesis reactions, including the asymmetric hydrogenation of aromatics which is useful for producing optically active cyclic compounds.

## Claims

1. A ruthenium-diphosphine-carboxylate complex represented by the following general formula (1). 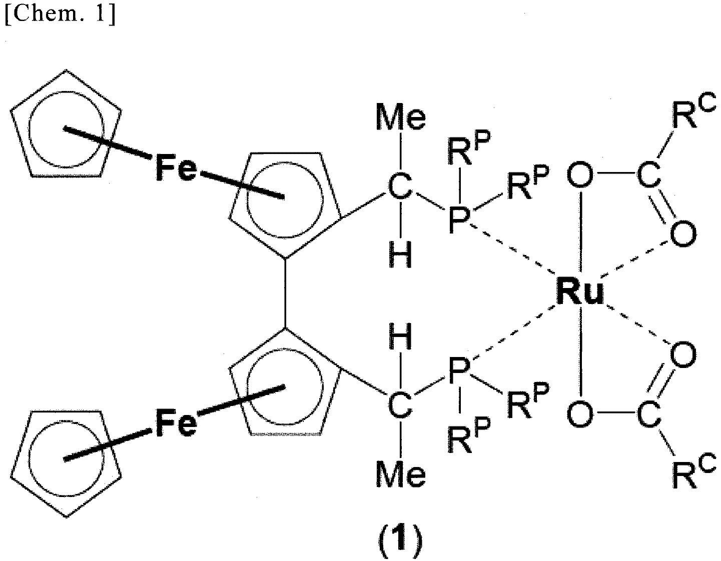 [Wherein,
a solid line represents a single bond, a double line represents a double bond, and a dashed line represents a coordinate bond;
H represents a hydrogen atom, C represents a carbon atom, O represents an oxygen atom, and P represents a phosphorus atom;
Me represents a methyl group;
Fe represents a divalent iron ion, a pentagon containing a circle represents a cyclopentadienyl anion, and a bold line represents six-electron donation of the cyclopentadienyl anion to Fe;
R^{P} represents a group selected from the group consisting of an alkyl group, a cycloalkyl group, a heteroaryl group, and an aryl group which may have a substituent;
Ru represents a divalent ruthenium ion;
R^{C} represents a group selected from the group consisting of an alkyl group, a halogenoalkyl group, a cycloalkyl group, and an aryl group.]

2. The ruthenium-diphosphine-carboxylate complex according to claim 1, which is an optically active form.

3. The ruthenium-diphosphine-carboxylate complex according to claim 1 or 2, wherein R^{P} is an aryl group which may have a substituent.

4. The ruthenium-diphosphine-carboxylate complex according to any one of claim 1 to 3, wherein R^{C} is selected from the group consisting of an alkyl group and a cycloalkyl group.

5. A method for producing the ruthenium-diphosphine-carboxylate complex according to any one of claim 1 to 4, comprising reacting a diphosphine compound represented by the following general formula (2) 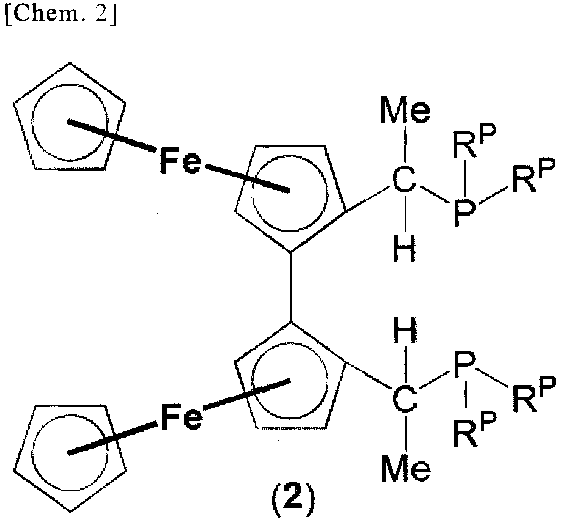 [Wherein,
a solid line represents a single bond;
H represents a hydrogen atom, C represents a carbon atom, and P represents
a phosphorus atom;
Me represents a methyl group;
Fe represents a divalent iron ion, a pentagon containing a circle represents a cyclopentadienyl anion, and a bold line represents six-electron donation of the cyclopentadienyl anion to Fe;
R^{P} represents a group selected from the group consisting of an alkyl group, a cycloalkyl group, a heteroaryl group, and an aryl group which may have a substituent.]
with a ruthenium-alkylbenzene-carboxylate complex represented by the following general formula (3). 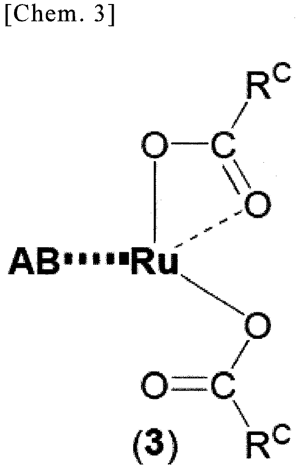
[Wherein,
a solid line represents a single bond, a double line represents a double bond, and a dashed line represents a coordinate bond;
C represents a carbon atom, and O represents an oxygen atom;
Ru represents a divalent ruthenium ion, AB represents an alkylbenzene, and a bold dashed line represents six-electron donation of the alkylbenzene to Ru;
R^{C} represents a group selected from the group consisting of an alkyl group, a halogenoalkyl group, a cycloalkyl group, and an aryl group.]

6. The method according to claim 5, wherein the diphosphine compound represented by the general formula (2) is an optically active form.

7. The method according to claim 5 or 6, wherein R^{P} in the general formula (2) is an aryl group which may have a substituent.

8. The method according to any one of claim 5 to 7, wherein R^{C} in the general formula (3) is selected from the group consisting of an alkyl group and a cycloalkyl group.

9. The method according to any one of claim 5 to 8, wherein AB in the general formula (3) is selected from the group consisting of benzene, 1,3,5-trimethylbenzene, 1-methyl-4-isopropylbenzene, and hexamethylbenzene.
